(19) Europäisches Patentamt / European Patent Office / Office européen des brevets

(11) EP 2 931 377 B1

(12) **EUROPÄISCHE PATENTSCHRIFT**

(45) Veröffentlichungstag und Bekanntmachung des Hinweises auf die Patenterteilung:
**05.07.2017 Patentblatt 2017/27**

(21) Anmeldenummer: **13795211.5**

(22) Anmeldetag: **20.11.2013**

(51) Int Cl.:
***A61Q 5/00*** (2006.01) ***A61K 8/894*** (2006.01)
***A61K 8/60*** (2006.01) ***A61K 8/27*** (2006.01)
***A61K 8/23*** (2006.01) ***A61K 8/49*** (2006.01)
***A61K 8/41*** (2006.01) ***A61K 8/42*** (2006.01)
***A61K 8/44*** (2006.01) ***A61K 8/58*** (2006.01)
***A61K 8/64*** (2006.01) ***A61K 8/73*** (2006.01)
***A61K 8/92*** (2006.01) ***A61K 8/97*** (2017.01)

(86) Internationale Anmeldenummer:
**PCT/EP2013/074212**

(87) Internationale Veröffentlichungsnummer:
**WO 2014/090520 (19.06.2014 Gazette 2014/25)**

(54) **HAARPFLEGEMITTEL MIT ANTISCHUPPENMITTELN UND AUSGEWÄHLTEN SILIKONEN ENTHALTEND ZUCKERSTRUKTUREN**

HAIR CARE PRODUCTS WITH ANTI DANDRUFF AGENTS AND SELECTED SILICONES CONTAINING SUGAR STRUCTURES

PRODUITS DE SOINS CHEVEUX AVEC MOYENS ANTIPELLICULAIRES ET SILICONES CHOISIS CONTENANT DES STRUCTURES DE SUCRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.12.2012 DE 102012222771**

(43) Veröffentlichungstag der Anmeldung:
**21.10.2015 Patentblatt 2015/43**

(73) Patentinhaber: **Henkel AG & Co. KGaA**
**40589 Düsseldorf (DE)**

(72) Erfinder:
- **METTE, Manuela**
  **23923 Kleinfeld (DE)**
- **HARTWICH, Christa**
  **25337 Elmshorn (DE)**
- **KAHRE, Jörg**
  **42799 Leichlingen (DE)**

(56) Entgegenhaltungen:
**WO-A1-2006/012930** **WO-A1-2010/089228**
**WO-A2-98/49998** **WO-A2-2010/075913**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 931 377 B1

## Beschreibung

Die vorliegende Erfindung beschreibt Zusammensetzungen zur Behandlung von keratinischen Fasern, insbesondere menschlichen Haaren, welche neben den konditionierenden Eigenschaften sowie dem Glanz und der Haptik von keratinischen Fasern, insbesondere menschlichen Haaren, gegenüber Zusammensetzungen des Standes der Technik eine verbesserte Wirkung in Bezug auf die Entfernung von Schuppen auf den keratinischen Fasern zeigt.

Ein wichtiger Aspekt bei der Haar- und Kopfhautbehandlung stellt die Bekämpfung von Schuppen dar. Im Allgemeinen wird unter Anti-Schuppen-Behandlung die Bekämpfung des Phytosporum Ovale-Hefepilzes verstanden, der bei übermäßigem Auftreten ursächlich ist für kosmetische Schuppen. Als wirksame Antischuppenwirkstoffe haben sich Zink-Pyrithion, Climbazol, Octopirox, Ketoconazol, Selendisulfid, selenhaltige Pflanzenöle und Extrakte bewährt.

Möglicherweise aufgrund der Ausbildung von Resistenzen oder durch weitere Einflüsse wie Stress, Ernährung und weiteren Haarbehandlungen, insbesondere oxidativen Haarbehandlungen, zeigt sich immer häufiger, dass der Verbraucher mit den zur Verfügung stehenden Antischuppenmitteln nicht zufrieden ist. Dies kann auf umweltbedingte Einflüsse und häufige oxidative Haarbehandlungen zurückzuführen sein. Diese Einflüsse führen häufig zu verschlechterten Kämmbarkeiten der trockenen und der nassen keratinischen Fasern, insbesondere menschlichen Haaren und einem verschlechterten Glanz. Eine weitere Folge von wiederholten Behandlungen keratinischer Fasern mit tensidischen und/oder oxidativen Mitteln ist ein starkes Nachfetten der keratinischen Fasern sowie eine starke Neigung zur vermehrten Bildung von Kopfhautschuppen. Die Aufgabe der vorliegenden Erfindung besteht daher darin, neue Haarbehandlungsmittel mit einer gesteigerten Wirksamkeit gegen Schuppen zur Verfügung zu stellen, welche die geschilderten Nachteile des Standes der Technik nicht aufweisen.

Es wurde nun überraschenderweise gefunden, dass die Aufgabe in hervorragendem Maße durch ein Haarbehandlungsmittel, welches einen Wirkstoffkomplex enthaltend als wesentliche Inhaltsstoffe mindestens einen Antischuppenwirkstoff und mindestens ein ausgewähltes kationisches Silikon enthält, gelöst wird.

Haarbehandlungsmittel enthaltend diesen Wirkstoffkomplex führen zur Verbesserung der Avivage, zur Verbesserung des Glanzes, zur Verbesserung des Feuchtehaushaltes sowie zum Schutz vor oxidativen Schädigungen und dem Verhindern des Nachfettens der keratinischen Fasern sowie zur Erhöhung der Waschbeständigkeit gefärbter keratinischer Fasern, insbesondere menschlicher Haare und insbesondere zur Verringerung der Bildung von Schuppen.

Ein erster Gegenstand der vorliegenden Erfindung ist daher ein Haarbehandlungsmittel enthaltend in einem geeigneten kosmetischen Träger - jeweils bezogen auf die gesamte Zusammensetzung des Mittels -

a) mindestens einen Antischuppenwirkstoff in einer Gesamtmenge von 0,01 bis 10,0 Gew.%, der ausgewählt sein muß aus

i. Zink-Pyrithion,
ii. Climbazol,
iii. Octopirox,
iv. Ketoconazol,
v. Selendisulfid,
vi. selenhaltige Pflanzenöle und
vii. selenhaltige Pflanzenextrakte sowie deren Mischungen und

b) mindestens ein Silikon enthaltend Zuckerstrukturen der folgenden Formel,

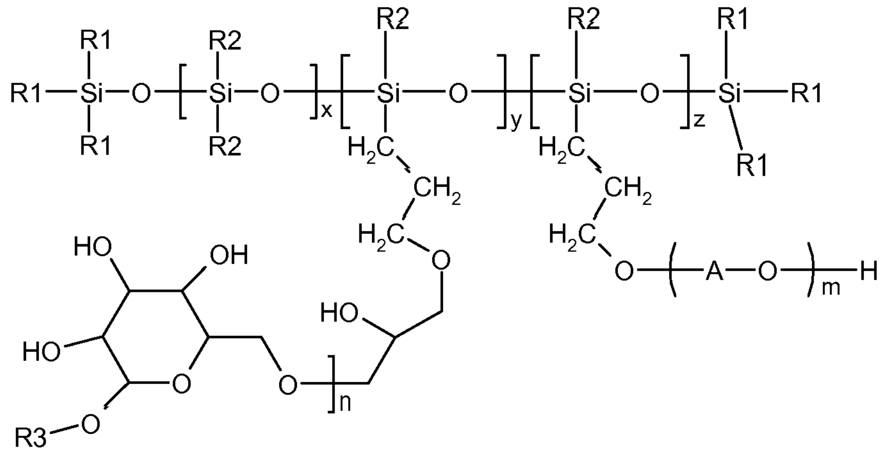

in

welcher A steht für eine Gruppe ausgewählt aus -CH2CH2-,-CH2CH2CH2- oder - CH2CH2CH2CH2- oder Mischungen dieser Gruppen, die Reste R1, R2 und R3 unabhängig voneinander stehen für eine Methyl, Ethyl, Propyl, iso-Propyl, Hydroxy, Methoxy oder Ethoxygruppe, x, y und z stehen für jeweils für eine ganze Zahl von 1 bis 1000, n und m stehen unabhängig voneinander jeweils für eine ganze Zahl von 1 bis 100, in einer Gesamtmenge von 0,01 bis 5,0 Gew.%.

Beim Einsatz dieser Kombination kommt es zu überraschend guten Eigenschaften des behandelten Haares, insbesondere zu verbesserten Kämmbarkeiten, zu verbessertem Glanz und zu einer verbesserten Elastizität als auch zu einer deutlich gesteigerten Waschbeständigkeit gefärbten Haares, sowie zu einer längeren Haltbarkeit bei einer gleichzeitigen besseren Umformleistung bei Wellvorgängen wie Wasserwelle und Dauerwelle. Insbesondere jedoch wird das Auftreten von Kopfhautschuppen durch diese Zusammensetzung deutlich verzögert. Haarbehandlungsmittel im Sinne der vorliegenden Erfindung sind beispielsweise Haarshampoos, Haarkonditionierer, konditionierende Shampoos, Haarspülungen, Haarkuren, Haarpackungen, Haar-Tonics, Haarfärbeshampoos oder deren Kombinationen. Insbesondere werden das Haar konditionierende Zusammensetzungen wie Haarspülungen, Haarkuren, Haarpackungen, Haaröle und- lotionen sowohl als leave-on, also als auf dem Haar bis zur nächsten Haarwäsche verbleibende Produkte, als auch als rinse-off, also wenige Sekunden bis wenige Stunden nach der Anwendung wieder auszuspülende Produkte, unter den erfindungsgemäßen Haarbehandlungsmitteln verstanden.

Unter Kämmbarkeit versteht sich erfindungsgemäß sowohl die Kämmbarkeit der nassen Faser, als auch die Kämmbarkeit der trockenen Faser.

Als Griff definiert sich die Taktilität eines Faserkollektivs, wobei der Fachmann sensorisch die Parameter Fülle und Geschmeidigkeit des Kollektivs fühlt und bewertet.

Unter Formgebung wird die Fähigkeit verstanden, einem Kollektiv zuvor behandelter keratinhaltiger Fasern, insbesondere menschlicher Haare, eine Formänderung zu verleihen. In der Haarkosmetik wird auch von Frisierbarkeit gesprochen.

Unter Restrukturierung im Sinne der Erfindung, ist eine Verringerung der durch verschiedenartigste Einflüsse entstandenen Schädigungen keratinischer Fasern zu verstehen. Hierbei spielt beispielsweise die Wiederherstellung der natürlichen Festigkeit eine wesentliche Rolle. Restrukturierte Fasern zeichnen sich durch einen verbesserten Glanz, durch einen verbesserten Griff und durch eine leichtere Kämmbarkeit aus. Zusätzlich weisen sie eine verbesserte Festigkeit und Elastizität auf. Ferner läßt sich eine erfolgreiche Restrukturierung physikalisch als Schmelzpunktserhöhung im Vergleich zur geschädigten Faser nachweisen. Je höher der Schmelzpunkt des Haares ist, desto fester ist die Struktur der Faser.

Unter Waschechtheit im Sinne der Erfindung ist die Erhaltung der ursprünglichen Färbung hinsichtlich Nuance und/oder Intensität zu verstehen, wenn die keratinische Faser dem wiederholten Einfluss von wässrigen Mitteln, insbesondere tensidhaltigen Mitteln wie Shampoos, ausgesetzt wird.

Weiterhin zeichnen sich die erfindungsgemäßen Zusammensetzungen enthaltend den erfindungsgemäßen Wirkstoffkomplex durch einen deutlich verbesserten Zustand der keratinischen Fasern in Bezug auf den Feuchtehaushalt der keratinischen Fasern aus. Weiterhin führt der erfindungsgemäße Wirkstoffkomplex zu einem deutlichen Schutz der keratinischen Fasern vor Hitzeeinwirkungen, beispielsweise beim Föhnen keratinischer Fasern. Der Schutz der Oberfläche von keratinischen Fasern vor Hitzeeinwirkung ist insbesondere bei der Verwendung von Glätteisen oder Haartrocknern von großer Bedeutung. Schließlich wurde überraschender Weise festgestellt, dass die erfindungsgemäßen Zusammensetzungen zu einer deutlich verzögerten Wiederanschmutzung der keratinischen Fasern führen.

Ein wässriger kosmetischer Träger enthält mindestens 50 Gew.-% Wasser.

Unter wässrig-alkoholischen kosmetischen Trägern sind im Sinne der vorliegenden Erfindung wässrige Lösungen enthaltend 3 bis 70 Gew.-% eines $C_1$-$C_6$-Alkohols, insbesondere Methanol, Ethanol bzw. Propanol, Isopropanol, Butanol, Isobutanol, tert.-Butanol, n-Pentanol, iso-Pentanole, n-Hexanol, iso-Hexanole, Glykol, Glycerin, 1,2-Pentandiol, 1,5-Pentandiol, 1,2-Hexandiol oder 1,6-Hexandiol zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel. Besonders bevorzugt ist Wasser.

Der erste erfindungsgemäße Inhaltsstoff a) ist ein Antischuppenwirkstoff. Erfindungsgemäße Antischuppenwirkstoffe sind:

i. Zink-Pyrithion,
ii. Climbazol,
iii. Octopirox,
iv. Ketoconazol,
v. Selendisulfid,
vi. selenhaltige Pflanzenöle und

vii. selenhaltige Pflanzenextrakte sowie deren Mischungen.

Mit besonderem Vorzug werden Zink-Pyrithion, Climbazol, Octopirox und / oder Ketoconazol sowie deren Mischungen in den erfindungsgemäßen Haarbehandlungsmitteln verwendet. Höchst bevorzugt ist die Verwendung von Zink-Pyrithion, Climbazol und Octopirox sowie der Mischung aus diesen, allerhöchst bevorzugt ist die Mischung aus Zink-Pyrithion, Climbazol und Octopirox.

Die erfindungsgemäßen Haarbehandlungsmittel enthalten mindestens einen Antischuppenwirkstoff wie zuvor beschrieben bevorzugt in einer Menge von 0,01 bis 10,0 Gew.-%, besonders bevorzugt von 0,05 bis 7,0 Gew.-%, ganz besonders bevorzugt von 0,1 bis 5,0 Gew.-%, jeweils bezogen auf das Gewicht des anwendungsbereiten Haarbehandlungsmittels.

Die zweite zwingende Komponente des Wirkstoffkomplexes ist Silikon enthaltend Zuckerstrukturen der folgenden Formel:.

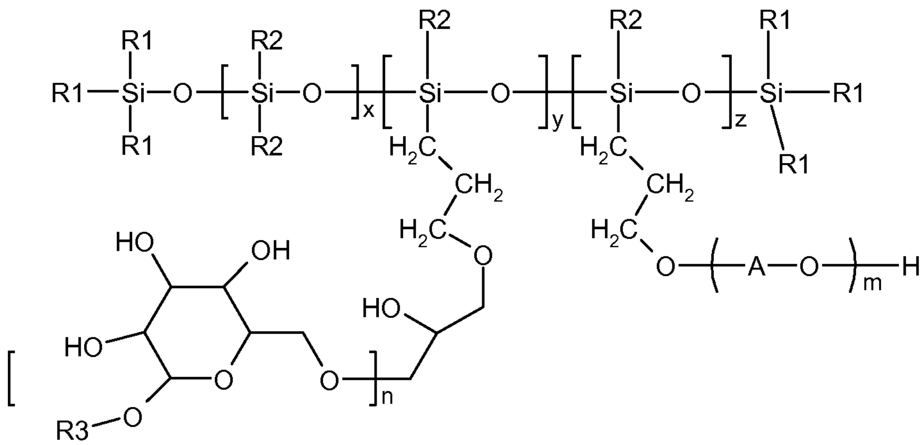

in welcher die Reste R1, R2 und R3 unabhängig voneinander stehen für eine Methyl, Ethyl, Propyl, iso-Propyl, Hydroxy, Methoxy oder Ethoxygruppe, x, y und z stehen für jeweils für eine ganze Zahl von 1 bis 1000, n und m stehen unabhängig voneinander jeweils für eine ganze Zahl von 1 bis 100. Bevorzugt stehen die Reste R1 unabhängig voneinander für Methyl, Hydroxy oder Methoxy, besonders bevorzugt für Methyl. Bevorzugt stehen die Reste R2 und R3 unabhängig voneinander für Methyl, Ethyl, Propyl oder iso-Propyl, besonders bevorzugt für Methyl. Bevorzugt stehen x, y und z jeweils unabhängig voneinander für eine ganze Zahl 1 bis 500 und besonders bevorzugt von 1 bis 200. Bevorzugt stehen n und m unabhängig voneinander für ganze Zahlen von 1 bis 50. Besonders bevorzugt steht m für eine ganze Zahl von 1 bis 20, höchst bevorzugt von 1 bis 15, und insbesondere für die Zahlen 2, 3, 4, 5, 6, 7, 8, 9, 10, 11, 12, 13, 14, 15. Besonders bevorzugt steht n für eine ganze Zahl von 1 bis 20, höchst bevorzugt für 1 bis 10 und insbesondere für eine Zahl 1, 2, 3, 4, 5, 6, 7, 8, 9, 10. A steht für eine Gruppe ausgewählt aus -CH2CH2-, -CH2CH2CH2- oder -CH2CH2CH2CH2- oder Mischungen dieser Gruppen, bevorzugt ausgewählt aus -CH2CH2-, - CH2CH2CH2- und deren Mischungen, höchst bevorzugt -CH2CH2-.

Ein höchst bevorzugtes Silikon der zuvor beschriebenen Formel enthaltend Zuckerstrukturen entspricht der im folgenden dargestellten Formel:

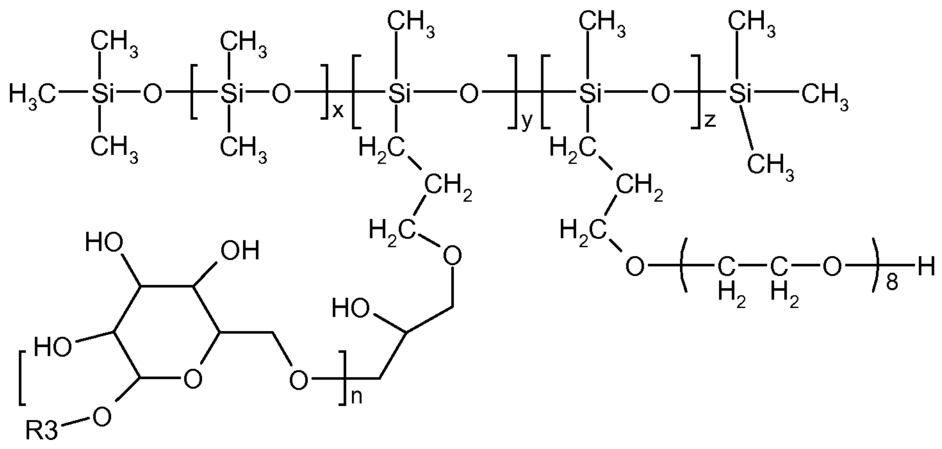

in welcher R3 die wie zuvor beschriebene Bedeutung hat und höchst bevorzugt für Methyl steht, x, y und z sowie n und m die bereits zuvor beschriebene Bedeutung haben.

Derartige Produkte sind unter der Handelsbezeichnung PolySuga Sil von der Fa. Colonial im Handel erhältlich. Ein besonders bevorzugtes Aminosilikon ist unter den Handelsbezeichnungen Poly Suga® Sil C-35P und/oder Poly Suga Sil® C-800P erhältlich. Das höchst bevorzugte Silikon enthaltend Zuckerstrukturen trägt die INCI - Bezeichnung PEG-8 PG-Coco-Glucoside Dimethicone.

Diese Zucker haltigen Silikonpolymere sind in den erfindungsgemäßen Zusammensetzungen in Mengen von 0,01 bis 5 Gew.%, bevorzugt in Mengen von 0,05 bis 5 Gew.% und ganz besonders bevorzugt in Mengen von 0,1 bis 5 Gew.% enthalten. Die allerbesten Ergebnisse werden dabei mit Mengen von 0,1 bis 2,5 Gew% jeweils bezogen auf die Gesamtzusammensetzung des jeweiligen Mittels enthalten.

Erfindungsgemäß bevorzugt ist es, wenn die erfindungsgemäßen Zusammensetzungen weiterhin mindestens eine quaternäre Verbindung enthalten. Die Wirksamkeit des erfindungsgemäßen Mittels wird dadurch nochmals gesteigert und die Stabilität der Zusammensetzung erheblich gefördert. In einer besonders bevorzugten Ausführungsform der vorliegenden Erfindung werden daher bevorzugt weiterhin ausgewählte quaternäre Ammoniumverbindungen mit den bereits zuvor beschriebenen zwingenden Inhaltsstoffen verwendet.

Quaternäre Ammoniumverbindungen sind prinzipiell monomere kationische oder amphotäre Ammoniumverbindungen, monomere Amine, Aminoamide, polymere kationische Ammoniumverbindungen sowie polymere amphotere Ammoniumverbindungen. Aus dieser Vielzahl an möglichen quaternären Ammoniumverbindungen haben sich die folgenden Gruppen als besonders geeignet erwiesen und werden jeweils für sich genommen in einer Menge von 0,1 bis 15,0 Gew.% eingesetzt. Diese Menge wird auch nicht unter- oder überschritten, wenn eine Mischung unterschiedlicher Verbindungen der quaternären Ammoniumverbindungen verwendet wird.

Kationische Tenside der Formel (Tkat1-1) bilden die erste Gruppe kationischer Tenside.

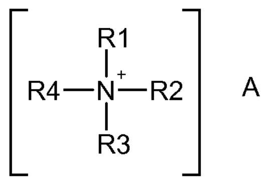

(Tkat1)

In der Formel (Tkat1) stehen R1, R2, R3 und R4 jeweils unabhängig voneinander für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann. A steht für ein physiologisch verträgliches Anion, beispielsweise Halogenide wie Chlorid oder Bromid sowie Methosulfate.

Beispiele für Verbindungen der Formel (Tkat1) sind Lauryltrimehtylammoniumchlorid, Cetyltrimethylammoniumchlorid, Cetyltrimethylammoniumbromid, Cetyltrimethylammonium-methosulfat, Dicetyldimethylammoniumchlorid, Tricetylmethylammoniumchlorid, Stearyltrimethylammoniumchlorid, Distearyldimethylammoniumchlorid, Lauryldimethylbenzylammoniumchlorid, Behenyltrimethylammoniumchlorid, Behenyltrimethylammoniumbromid, Behenyltrimethylammoniummethosulfat.

Esterquats gemäß der Formel (Tkat2) bilden eine bevorzugte Gruppe.

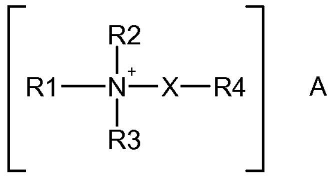

(Tkat2)

Hierin sind die Reste R1, R2 und R3 jeweils unabhängig voneinander und können gleich oder verschieden sein. Die Reste R1, R2 und R3 bedeuten:

- ein verzweigter oder unverzweigter Alkylrest mit 1 bis 4 Kohlenstoffatomen, welcher mindestens eine Hydroxylgruppe enthalten kann, oder
- ein gesättigter oder ungesättigter, verzweigter oder unverzweigter oder ein cyclischer gesättigter oder ungesättigter Alkylrest mit 6 bis 30 Kohlenstoffatomen, welcher mindestens eine Hydroxylgruppe enthalten kann, oder
- ein Aryl oder Alkarylrest, beispielsweise Phenyl oder Benzyl,
- den Rest (- X - R4), mit der Maßgabe, daß höchstens 2 der Reste R1, R2 oder R3 für diesen Rest stehen können:

Der Rest -(X - R4) ist mindestens 1 bis 3 mal enthalten.

Hierin steht X für:

1) -(CH2)n- mit n = 1 bis 20, vorzugsweise n = 1 bis 10 und besonders bevorzugt n = 1 - 5, oder
2) -(CH2-CHRS-O)n- mit n = 1 bis 200, vorzugsweise 1 bis 100, besonders bevorzugt 1 bis 50, und besonders bevorzugt 1 bis 20 mit R5 in der Bedeutung von Wasserstoff, Methyl oder Ethyl,
3) eine Hydroxyalkylgruppe mit ein bis vier Kohlenstoffatomen, welche verzweigt oder unverzweigt sein kann, und welche mindestens eine und höchstens 3 Hydroxygruppen enthält. Beispiele sind: $-CH_2OH$, $-CH_2CH_2OH$, -CHOH-CHOH, $-CH_2CHOHCH_3$, $-CH(CH_2OH)_2$, $-COH(CH_2OH)_2$, $-CH_2CHOHCH_2OH$, $-CH_2CH_2CH_2OH$ und Hydroxybutylreste,

und R4 steht für:

1) R6-O-CO-, worin R6 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, oder
2) R7-CO-, worin R7 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann,

und A steht für ein physiologisch verträgliches organisches oder anorganisches Anion und wird an dieser Stelle stellvertretend für alle auch im Folgenden beschriebenen Strukturen definiert. Das Anion aller beschriebenen kationischen Verbindungen ist ausgewählt aus den Halogenidionen, Fluorid, Chlorid, Bromid, lodid, Sulfaten der allgemeinen Formel $RSO_3^-$, worin R die Bedeutung von gesättigtem oder ungesättigtem Alkylresten mit 1 bis 4 Kohlenstoffatomen hat, oder anionischen Reste organischer Säuren wie Maleat, Fumarat, Oxalat, Tartrat, Citrat, Lactat oder Acetat.

Solche Produkte werden beispielsweise unter den Warenzeichen Rewoquat®, Stepantex®, Dehyquart®, Armocare® und Akypoquat® vertrieben. Die Produkte Armocare® VGH-70, Dehyquart® F-75, Dehyquart® C-4046, Dehyquart® L80, Dehyquart® F-30, Dehyquart® AU-35, Rewoquat® WE18, Rewoquat® WE38 DPG, Stepantex® VS 90 und Akypoquat® 131 sind Beispiele für diese Esterquats.

Weitere erfindungsgemäß besonders bevorzugte Verbindungen der Formel (Tkat1-2) zählen zur Formel (Tkat2.1), den kationischen Betainestern.

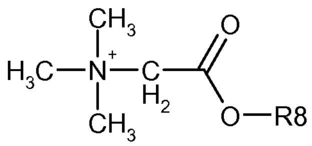

(Tkat2.1)

R8 entspricht in seiner Bedeutung R7.

Besonders bevorzugt sind die Esterquats mit den Handelsbezeichnungen Armocare® VGH-70, sowie Dehyquart® F-75, Dehyquart® L80, Stepantex® VS 90 und Akypoquat® 131.

In bevorzugten erfindungsgemäßen Mitteln werden kationische Tenside der Formel (bl) innerhalb engerer Mengenbereiche eingesetzt, so daß bevorzugte erfindungsgemäße Haarbehandlungsmittel dadurch gekennzeichnet sind, daß sie 0,1 bis 15 Gew.-%, vorzugsweise 0,5 bis 10 Gew.-%, weiter bevorzugt 1 bis 10 Gew.-%, noch weiter bevorzugt 1,5 bis 10 Gew.-% und insbesondere 2 bis 5 Gew.-% mindestens einer Verbindung der allgemeinen Formel (I) enthalten.

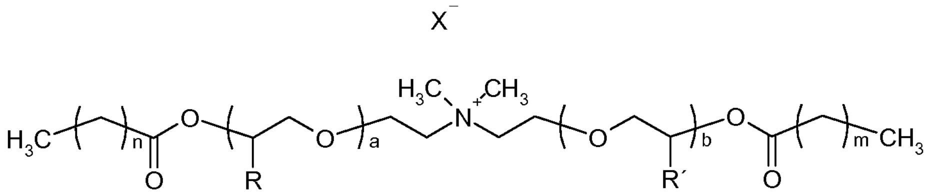

Formel (I)

in der

- n und m: unabhängig voneinander für ganze Zahlen zwischen 5 und 40 stehen, mit der Maßgabe, daß $n + m \geq 38$ ist; besonders bevorzugt ist n = m; höchst bevorzugt ist n = m = 20.
- a und b: unabhängig voneinander für ganze Zahlen zwischen 1 und 10 stehen; besonders unabhängig voneinander für 1, 2, 3, 4 oder 5 stehen, bevorzugt gilt hierbei die Gleichung $a + 2 \geq b \geq a - 2$ und höchst bevorzugt ist a = b = 3.
- R und R’: unabhängig voneinander ausgewählt sind aus -H und $-CH_3$; bevorzugt gilt R = R’, so daß vorzugsweise entweder PEG- oder PPG-Diesterquats eingesetzt werden; ganz besonders bevorzugt gilt R = R’ = $-CH_3$
- X-: ein physiologisch verträgliches Anion, ein Halogenid wie Chlorid, Bromid oder Iodid, Toluolsulfonat, Methosulfat usw., und besonders bevorzugt Methosulfat ist.

Insbesondere bei der Verwendung einer der Verbindungen der Formel (I) wie zuvor beschrieben, hat es sich gezeigt, daß die Pflegeeffekte der erfindungsgemäßen Mittel weiter gesteigert und insbesondere die Stabilität der Mittel weiter verbessert werden kann, wenn die Mittel zusätzlich zu der bzw. den Verbindung(en) der Formel (I) bestimmte acylierte Diamine enthalten.

Erfindungsgemäße bevorzugte Haarbehandlungsmittel sind daher dadurch gekennzeichnet, daß sie zusätzlich 0,1 bis 10 Gew.-% mindestens einer Verbindung der Formel (II) enthalten

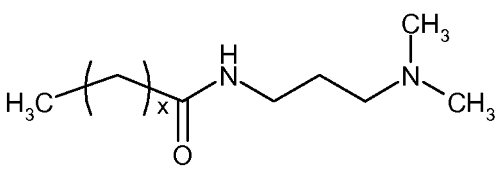

(II),

in der x für 18, 19, 20, 21, 22, 23 oder 24 steht.

Verbindungen der Formel (II) mit n = 20 sind dabei besonders bevorzugt. Höchst bevorzugte erfindungsgemäße Mittel zeichnen sich dadurch aus, dass sie eine Verbindung der Formel (I) immer gemeinsam mit einer Verbindung der allgemeinen Formel (II) enthalten.

Eine weitere Gruppe sind quartäre Imidazolinverbindungen. Die im Folgenden dargestellte Formel (Tkat2) zeigt die Struktur dieser Verbindungen.

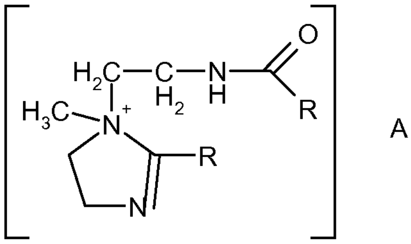

(Tkat2)

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel (Tkat2) enthalten für R jeweils den gleichen Kohlenwasserstoffrest. Die Kettenlänge der Reste R beträgt bevorzugt 12 bis 21 Kohlenstoffatome. A steht für ein Anion wie zuvor beschrieben. Besonders erfindungsgemäße Beispiele sind beispielsweise unter den INCII - Bezeichnungen Quaternium-27, Quaternium-72, Quaternium-83 und Quaternium-91 erhältlich. Höchst bevorzugt ist erfindungsgemäß Quaternium-91.

In einer besonders bevorzugten Ausführungsform der Erfindung enthalten die erfindungsgemäßen Mittel weiterhin mindestens ein Amin und/oder kationisiertes Amin, insbesondere ein Amidoamin und/oder ein kationisiertes Amidoamin mit den folgenden Strukturformeln:

$$R1 - NH - (CH_2)_n - N^+R^2R^3R^4 \; A \qquad (Tkat3)$$

worin R1 ein Acyl-oder Alkylrest mit 6 bis 30 C-Atomen, welche verzweigt oder unverzweigt, gesättigt oder ungesättigt sein können, und wobei der Acylrest und/oder der Alkylrest mindestens eine OH-Gruppe enthalten können, und R2, R3 und R4 jeweils unabhängig voneinander

1) Wasserstoff oder
2) ein Alkylrest mit 1 bis 4 C-Atomen, welcher gleich oder verschieden, gesättigt oder ungesättigt sein kann, und
3) eine verzweigte oder unverzweigte Hydroxyalkylgruppe mit ein bis 4 Kohlenstoffatomen mit mindestens einer und höchstens drei Hydroxygruppen beispielsweise $-CH_2OH$, $-CH_2CH_2OH$, -CHOHCHOH, $-CH_2CHOHCH_3$, $-CH(CH_2OH)_2$, $-COH(CH_2OH)_2$, $-CH_2CHOHCH_2OH$, $-CH_2CH_2CH_2OH$ und Hydroxybutylreste, und

A ein Anion wie zuvor beschrieben und
n eine ganze Zahl zwischen 1 und 10 bedeuten.

Bevorzugt wird eine Zusammensetzung, in welcher das Amin und/oder das quaternisierte Amin gemäß allgemeiner Formeln (Tkat3) ein Amidoamin und/oder ein quaternisiertes Amidoamin ist, worin R1 ein verzweigter oder unverzweigter, gesättigter oder ungesättigter Acylrest mit 6 bis 30 C-Atomen, welcher mindestens eine OH-Gruppe enthalten kann, bedeutet. Bevorzugt ist hierbei ein Fettsäurerest aus Ölen und Wachsen, insbesondere aus natürlichen Ölen und Wachsen. Als Beispiele hierfür kommen Lanolin, Bienen-oder Candellilawachse in Betracht.

Bevorzugt sind auch solche Amidoamine und/oder quaternisierte Amidoamine, in denen R2, R3 und/oder R4 in der Formel (Tkat3) ein Rest gemäß der allgemeinen Formel $CH_2CH_2OR5$ bedeuten, worin R5 die Bedeutung von Alkylresten mit 1 bis 4 Kohlenstoffatomen, Hydroxyethyl oder Wasserstoff haben kann. Die bevorzugte Größe von n in der allgemeinen Formel (Tkat8) ist eine ganze Zahl zwischen 2 und 5.

Die Alkylamidoamine können sowohl als solche vorliegen und durch Protonierung in entsprechend saurer Lösung in eine quaternäre Verbindung in der Zusammensetzung überführt werden. Erfindungsgemäß bevorzugt sind die kationischen Alkylamidoamine.

Beispiele für derartige erfindungsgemäße Handelsprodukte sind Witcamine® 100, Incromine® BB, Mackine® 401und andere Mackine® -Typen, Adogen® S18V, und als permanent kationische Aminoamine: Rewoquat® RTM 50, Empigen® CSC, Swanol® Lanoquat DES-50, Rewoquat® UTM 50, Schercoquat® BAS, Lexquat® AMG-BEO, oder Incroquat® Behenyl HE.

Ein weiteres erfindungsgemäßes Fettsäureamid entspricht der allgemeinen Formel (I).

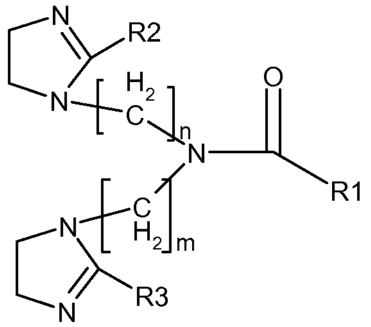

Formel (I)

in welcher R1, R2 und R3 unabhängig voneinander stehen für eine lineare verzweigte oder unverzweigte C6 bis C30, bevorzugt C8 bis C24, bevorzugter C12 bis C22 und höchst bevorzugt C12 bis C18 Alkyl- oder Alkenylgruppe. R1 bis R3 stehen vorzugsweise für Capryl, Caprylyl, Octyl, Nonyl, Decanyl, Lauryl, Myristyl, Cetyl, Stearyl, Isostearyl, Oleyl, Behenyl oder Arachidyl. Weiterhin gilt besonders bevorzugt R2 gleich R3 und höchst bevorzugt R1 gleich R2 gleich R3. Die Buchstaben n und m stehen unabhängig voneinander für ganze Zahlen von 1 bis 10, vorzugsweise 2 bis 6 und höchst bevorzugt für 2, 3 und/oder 4, wobei höchst bevorzugt n = m ist. Höchst bevorzugt sind R1 gleich R2 gleich R3 und ausgewählt aus Capryl, Caprylyl, Octyl, Nonyl, Decanyl, Lauryl, Myristyl, Cetyl, Stearyl, Isostearyl, Oleyl, Behenyl oder Arachidyl und n = m = 2. Am bevorzugtesten ist R1 = R2 = R3 und ausgewählt aus Lauryl, Myristyl, Cetyl, Stearyl, Isostearyl, Oleyl, Behenyl oder Arachidyl, worunter Cetyl, Stearyl, Isostearyl, Oleyl oder Behenyl besonders bevorzugt sind und n = m = 2. Die bevorzugteste Verbindung der Formel (I) ist diejenige, welche den INCI Namen Bis-Ethyl(isostearylimidazoline) Isostearamide trägt. Letztere Verbindung ist unter der Handelsbezeichnung Keradyn® HH von der Firma Croda im Handel erhältlich.

Die zuvor genannten kationischen Tenside können einzeln oder in beliebigen Kombinationen miteinander verwendet werden, wobei Mengen zwischen 0,01 bis 10 Gew.%, bevorzugt in Mengen von 0,01 bis 7,5 Gew.% und ganz besonders bevorzugt in Mengen von 0,1 bis 5,0 Gew.% enthalten. Die allerbesten Ergebnisse werden dabei mit Mengen von 0,1 bis 3,0 Gew.% jeweils bezogen auf die Gesamtzusammensetzung des jeweiligen Mittels erhalten.

Weitere quarternäre Ammoniumverbindungen sind kationische und amphotere Polymer.

Die kationischen und/oder amphoteren Polymere können Homo- oder Copolymere oder Polymere auf Basis natürlicher Polymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxy-alkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Aus der Vielzahl dieser Polymere haben sich als besonders wirkungsvolle Bestandteile des erfindungsgemäßen Wirkstoffkomplexes erwiesen:

Homopolymere der allgemeinen Formel $-\{CH_2-[CR^1COO-(CH_2)_mN^+R^2R^3R^4]\}_n\ X^-$,
in der $R^1$= -H oder $-CH_3$ ist, $R^2$, $R^3$ und $R^4$ unabhängig voneinander ausgewählt sind aus C1-4-Alkyl-, -Alkenyl- oder-Hydroxyalkylgruppen, m = 1, 2, 3 oder 4, n eine natürliche Zahl und
$X^-$ ein physiologisch verträgliches organisches oder anorganisches Anion ist. Im Rahmen dieser Polymere sind diejenigen erfindungsgemäß bevorzugt, für die mindestens eine der folgenden Bedingungen gilt: $R^1$ steht für eine Methylgruppe, $R^2$, $R^3$ und $R^4$ stehen für Methylgruppen, m hat den Wert 2.

Als physiologisch verträgliches Gegenionen $X^-$ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Methosulfate und Halogenidionen, insbesondere Chlorid.

Geeignete kationische Polymere sind beispielsweise Copolymere gemäß der Formel (Copo), welche in den erfindungsgemäßen Haarbehandlungsmitteln bevorzugt in einer Menge - bezogen auf ihr Gewicht - von 0,001 bis 5 Gew.-%, vorzugsweise 0,0025 bis 2,5 Gew.-%, besonders bevorzugt 0,005 bis 1 Gew.-%, weiter bevorzugt 0,0075 bis 0,75 Gew.-% und insbesondere 0,01 bis 0,5 Gew.-% enthalten sind.

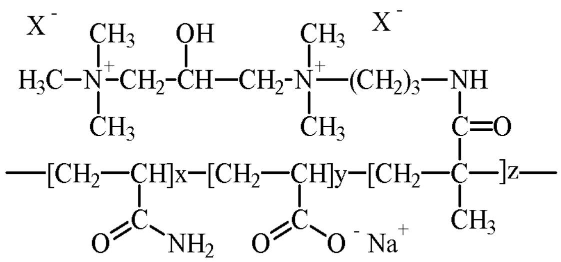

Formel (Copo)

in der gilt:

$$x + y + z = Q$$

Q steht für Werte von 3 bis 55000, vorzugsweise von 10 bis 25000, besonders bevorzugt von 50 bis 15000, weiter bevorzugt von 100 bis 10000, noch weiter bevorzugt von 500 bis 8000 und insbesondere von 1000 bis 5000,

x steht für (0 bis 0,5) Q, vorzugsweise für (0 bis 0,3) Q und insbesondere für die Werte 0, 1, 2, 3, 4, 5, wobei der Wert 0 bevorzugt ist,

y steht für (0,1 bis 0,95) Q, vorzugsweise für (0,5 bis 0,7) Q und insbesondere für Werte von 1 bis 24000, vorzugsweise von 5 bis 15000, besonders bevorzugt von 10 bis 10000 und insbesondere von 100 bis 4800,

z steht für (0,001 bis 0,5) Q, vorzugsweise für (0,1 bis 0,5) Q und insbesondere für Werte von 1 bis 12500, vorzugsweise von 2 bis 8000, besonders bevorzugt von 3 bis 4000 und insbesondere von 5 bis 2000.

Unabhängig davon, welche der bevorzugten Copolymere der Formel (Copo) eingesetzt werden, sind erfindungsgemäße Haarbehandlungsmittel bevorzugt, die dadurch gekennzeichnet sind, daß das Verhältnis von (y : z) 4:1 bis 1:2, vorzugsweise 4:1 bis 1:1 beträgt.

Unabhängig davon, welche Copolymere in den erfindungsgemäßen Mitteln eingesetzt werden, sind erfindungsgemäße Haarbehandlungsmittel bevorzugt, bei denen das Copolymer eine Molmasse von 10000 bis 20 Millionen $gmol^{-1}$, vorzugsweise von 100000 bis 10 Millionen $gmol^{-1}$, weiter bevorzugt von 500000 bis 5 Millionen $gmol^{-1}$ und insbesondere von 1,1 Millionen bis 2,2 Millionen $gmol^{-1}$ aufweist.

Ein höchst bevorzugtes Copolymer, welches wie zuvor dargestellt aufgebaut ist, ist unter der Bezeichnung Polyquaternium-74 im Handel erhältlich.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare® CTH (Cosmetic Rheologies) und Synthalen® CR (3V Sigma) im Handel erhältlich.

Das Homopolymer wird bevorzugt in Form einer nichtwäßrigen Polymerdispersion eingesetzt. Solche Polymerdispersionen sind unter den Bezeichnungen Salcare® SC 95 und Salcare® SC 96 im Handel erhältlich.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel G-O-B-N+$R_aR_bR_c$ $A^-$

G ist ein Anhydroglucoserest, beispielsweise Stärke- oder Celluloseanhydroglucose;
B ist eine divalente Verbindungsgruppe, beispielsweise Alkylen, Oxyalkylen, Polyoxyalkylen oder Hydroxyalkylen;
$R_a$, $R_b$ und $R_c$ sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in $R_a$, $R_b$ und $R_c$ vorzugsweise maximal 20 ist;
$A^-$ ist ein übliches Gegenanion und ist vorzugsweise Chlorid.

Kationische, also quaternisierte Cellulosen sind mit unterschiedlichem Substitutionsgrad, kationischer Ladungsdichte, Stickstoffgehalt und Molekulargewichten auf dem Markt erhältlich. Beispielsweise wird Polyquaternium-67 im Handel unter den Bezeichnungen Polymer® SL oder Polymer® SK (Amerchol) angeboten. Unter der Handelsbezeichnung Mirustyle® CP der Fa. Croda wird eine weitere höchst bevorzugte Cellulose angeboten. Diese ist eine Trimonium and Cocodimonium Hydroxyethylcellulose als derivatisierte Cellulose mit der INCI-Bezeichnung Polyquaternium-72. Polyquaternium-72 kann sowohl in fester Form als auch bereits in wässriger Lösung vorgelöst verwendet werden.

Weitere kationische Cellulosen sind Polymer JR® 400 (Amerchol, INCI-Bezeichnung Polyquaternium-10) sowie Polymer Quatrisoft® LM-200 (Amerchol, INCI-Bezeichnung Polyquaternium-24). Weitere Handelsprodukte sind die Verbindungen Celquat® H 100 und Celquat® L 200. Besonders bevorzugte kationische Cellulosen sind Polyquaternium-24, Polyquaternium-67 und Polyquaternium-72.

Geeignete kationische Guarderivate werden unter der Handelsbezeichnung Jaguar® vertrieben und haben die INCI-Bezeichnung Guar Hydroxypropyltrimonium Chloride. Weiterhin sind besonders geeignete kationische Guarderivate auch von der Fa. Hercules unter der Bezeichnung N-Hance® im Handel. Weitere kationische Guarderivate werden von der Fa. Cognis unter der Bezeichnung Cosmedia® vertrieben. Ein bevorzugtes kationisches Guarderivat ist das Handelsprodukt AquaCat® der Fa. Hercules. Bei diesem Rohstoff handelt es sich um ein bereits vorgelöstes kationisches Guarderivat. Die kationischen Guar-Derivate sind erfindungsgemäß bevorzugt.

Ein geeignetes Chitosan wird beispielsweise von der Firma Kyowa Oil& Fat, Japan, unter dem Handelsnamen Flonac® vertrieben. Ein bevorzugtes Chitosansalz ist Chitosoniumpyrrolidoncarboxylat, welches beispielsweise unter der Bezeichnung Kytamer® PC von der Firma Amerchol, USA, vertrieben wird. Weitere Chitosanderivate sind unter den Handelsbezeichnungen Hydagen® CMF, Hydagen® HCMF und Chitolam® NB/101 im Handel frei verfügbar.

Eine weitere Gruppe erfindungsgemäß hervorragend zu verwendender Polymere sind Polymere auf der Basis von Glucose. Die folgende Abbildung zeigt ein derartiges kationisches Alkyloligoglucosid.

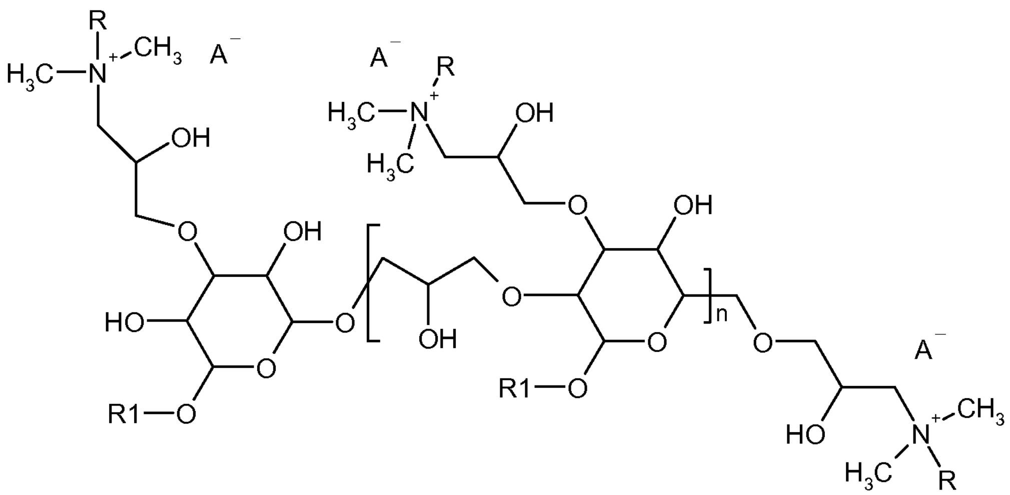

In der zuvor dargestellten Formel stehen die Reste R unabhängig voneinander für einen linearen oder verzweigten C6 bis C30 Alkylrest, einen linearen oder verzweigten C6 - C30 Alkenylrest, vorzugsweise steht der Rest R für einen Rest R ausgewählt aus: Lauryl, Myristyl, Cetyl, Stearyl, Oleyl, Behenyl oder Arachidyl.

Die Reste R1 stehen unabhängig voneinander für einen linearen oder verzweigten C6 bis C30 Alkylrest, einen linearen oder verzweigten C6 bis C30 Alkenylrest, vorzugsweise steht der Rest R für einen Rest ausgewählt aus: Butyl, Capryl, Caprylyl, Octyl, Nonyl, Decanyl, Lauryl, Myristyl, Cetyl, Stearyl, Oleyl, Behenyl oder Arachidyl. Besonders bevorzugt sind die Reste R1 gleich. Noch bevorzugter sind die Reste R1 ausgewählt aus technischen Mischungen der Fettalkoholschnitte aus C6/C8 - Fettalkoholen, C8/C10 - Fettalkoholen, C10/C12 - Fettalkoholen, C12/C14 - Fettalkoholen, C12 / C18 - Fettalkoholen, und höchst bevorzugt sind hierbei diejenigen technischen Fettalkoholschnitte, welche pflanzlichen Ursprunges sind. Das Gegenion zur kationischen Ladung ist ein physiologisch verträgliches Anion, beispielsweise Halogenid, Methosulfat, Phosphat, Citrat, Tartrat, etc. Bevorzugt ist das Gegenion ein Halogenid, wie Fluorid, Chlorid, Bromid oder Methosulfat. Höchst bevorzugt ist das Anion Chlorid.

Besonders bevorzugte Beispiele für die kationischen Alkyloligoglucoside sind die Verbindungen mit den INCI - Bezeichnungen Polyquaternium-77, Polyquaternium-78, Polyquaternium-79, Polyquaternium-80, Polyquaternium-81 und Polyquaternium-82. Höchst bevorzugt sind die kationischen Alkyloligoglucoside mit den Bezeichnungen Polyquaternium-77, Polyquaternium-81 und Polyquaternium-82.

Derartige Verbindungen können beispielsweise unter der Bezeichnung Poly Suga® Quat von der Fa. Colonial Chemical Inc. bezogen werden.

Die kationischen Alkyloligoglucoside werden in einer Gesamtmenge von 0,01 bis 10,0 Gew.%, vorzugsweise von 0,05 bis 5,0 Gew.%, noch bevorzugter von 0,1 bis 3,0 Gew.% und höchst bevorzugt in Mengen von 0,2 bis 2,0 Gew.% jeweils bezogen auf das Gesamtgewicht der Zusammensetzung verwendet. Erfindungsgemäß umfaßt ist selbstverständlich auch, daß mehr Mischungen von kationischen Alkyloligoglucosiden verwendet werden können. Bevorzugt ist in diesem Falle, wenn jeweils ein langkettiges und ein kurzkettiges kationisches Alkyloligoglucosid gleichzeitig verwendet werden.

Ein weiteres bevorzugtes kationisches Polymer kann auf der Grundlage von Ethanolamin erhalten werden. Das Polymer ist unter der Bezeichnung Polyquaternium-71 im Handel erhältlich.

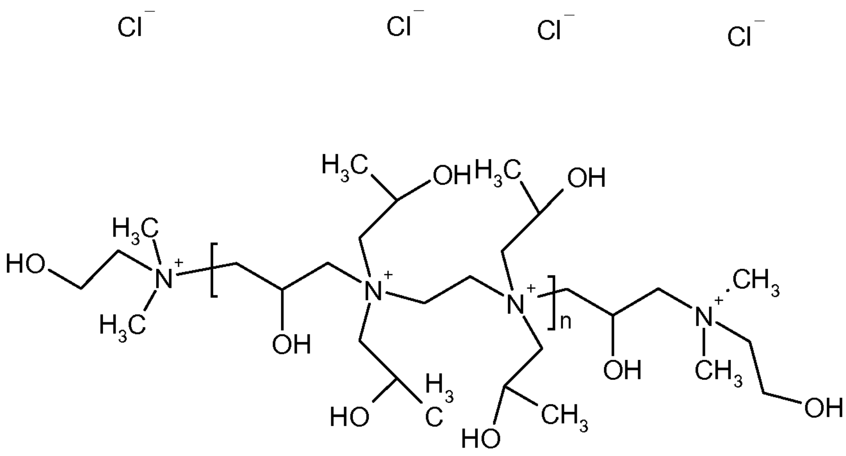

Dieses Polymer kann beispielsweise unter der Bezeichnung Cola® Moist 300 P von der Fa. Colonial Chemical Inc. bezogen werden.

Das Polyquaternium-71 wird in einer Gesamtmenge von 0,01 bis 10,0 Gew.%, vorzugsweise von 0,05 bis 5,0 Gew.%, noch bevorzugter von 0,1 bis 3,0 Gew.% und höchst bevorzugt in Mengen von 0,2 bis 2,0 Gew.% jeweils bezogen auf das Gesamtgewicht der Zusammensetzung verwendet. Weiterhin kann mit besonderem Vorzug ein kationisches Alkyloligoglucosid, wie in der folgenden Abbildung gezeigt, verwendet werden.

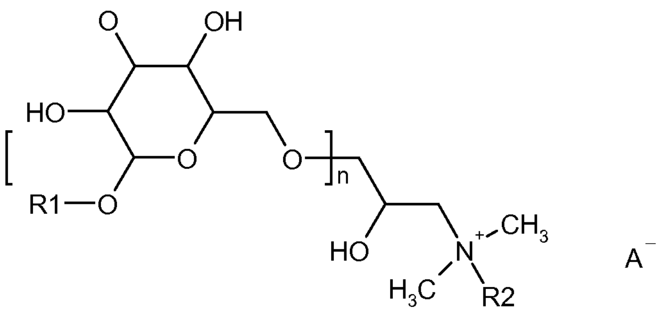

In der zuvor dargestellten Formel steht der Rest R2 für einen linearen oder verzweigten C6 bis C30 Alkylrest, einen linearen oder verzweigten C6 - C30 Alkenylrest, vorzugsweise steht der Rest R für einen Rest R ausgewählt aus: Lauryl, Myristyl, Cetyl, Stearyl, Oleyl, Behenyl oder Arachidyl. Der Rest R1 steht für einen linearen oder verzweigten C6 bis C30 Alkylrest, einen linearen oder verzweigten C6 bis C30 Alkenylrest, vorzugsweise steht der Rest R1 für einen Rest ausgewählt aus: Butyl, Capryl, Caprylyl, Octyl, Nonyl, Decanyl, Lauryl, Myristyl, Cetyl, Stearyl, Oleyl, Behenyl oder Arachidyl. Noch bevorzugter ist der Rest R1 ausgewählt aus technischen Mischungen der Fettalkoholschnitte aus C6/C8 - Fettalkoholen, C8/C10 - Fettalkoholen, C10/C12 - Fettalkoholen, C12/C14 - Fettalkoholen, C12/C18 - Fettalkoholen, und höchst bevorzugt sind hierbei diejenigen technischen Fettalkoholschnitte, welche pflanzlichen Ursprunges sind. Der Index n steht für eine Zahl zwischen 1 und 20, vorzugsweise zwischen 1 und 10, bevorzugter zwischen 1 und 5 und höchst bevorzugt zwischen 1 und 3. Das Gegenion zur kationischen Ladung, $A^-$, ist ein physiologisch verträgliches Anion, beispielsweise Halogenid, Methosulfat, Phosphat, Citrat, Tartrat, etc. Bevorzugt ist das Gegenion ein Halogenid, wie Fluorid, Chlorid, Bromid oder Methosulfat. Höchst bevorzugt ist das Anion Chlorid.

Besonders bevorzugte Beispiele für die kationischen Alkyloligoglucoside sind die Verbindungen mit den INCI - Bezeichnungen Laurdimoniumhydroxypropyl Decylglucosides Chloride, Laurdimoniumhydroxypropyl Laurylglucosides Chloride, Stearyldimoniumhydroxypropyl Decylglucosides Chloride, Stearyldimoniumhydroxypropyl Laurylglucosides Chloride, Stearyldimoniumhydroxypropyl Laurylglucosides Chloride oder Cocoglucosides Hydroxypropyltrimonium Chloride.

Derartige Verbindungen können beispielsweise unter der Bezeichnung Suga® Quat von der Fa. Colonial Chemical Inc. bezogen werden.

Die kationischen Alkyloligoglucoside werden in einer Gesamtmenge von 0,01 bis 10,0 Gew.%, vorzugsweise

von 0,05 bis 5,0 Gew.%, noch bevorzugter von 0,1 bis 3,0 Gew.% und höchst bevorzugt in Mengen von 0,2 bis 2,0 Gew.% jeweils bezogen auf das Gesamtgewicht der Zusammensetzung verwendet. Erfindungsgemäß umfaßt ist selbstverständlich auch, daß mehr Mischungen von kationischen Alkyloligoglucosiden verwendet werden können. Bevorzugt ist in diesem Falle, wenn jeweils ein langkettiges und ein kurzkettiges kationisches Alkyloligoglucosid gleichzeitig verwendet werden.

Ein besonders bevorzugtes erfindungsgemäßes kationisches Polymer ist das Copolymer aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle® 300 (28-32 Gew.-% Aktivsubstanz in Ethanol-Wasser-Gemisch, Molekulargewicht 350000) von der Firma ISP vertrieben wird.

Weitere bevorzugte kationische Polymere sind beispielsweise

- kationisierter Honig, beispielsweise das Handelsprodukt Honeyquat® 50,
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere mit der INCI - Bezeichnung Polyquaternium-7,
- Vinylpyrrolidon-Vinylimidazoliummethochlorid-Copolymere, wie sie unter den Bezeichnungen Luviquat® FC 370, FC 550 und der INCI-Bezeichnung Polyquaternium-16 sowie FC 905 und HM 552 angeboten werden,
- quaternisiertes Vinylpyrrolidon/Dimethylaminoethylmethacrylat, zum Beispiel Vinylpyrrolidon/Dimethylaminoethylmethacrylatmethosulfat Copolymer, das unter den Handelsbezeichnungen Gafquat® 755 N und Gafquat® 734 von der Firma Gaf Co., USA vertrieben wird und die INCI - Bezeichnung Polyquaternium-11,
- quaternierter Polyvinylalkohol,
- sowie die unter den Bezeichnungen Polyquaternium-2, Polyquaternium-17, Polyquaternium-18 und Polyquaternium-27 bekannten Polymeren mit quartären Stickstoffatomen in der Polymerhauptkette,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex® SF 40 angeboten werden.

Erfindungsgemäße amphotere Polymere sind solche Polymerisate, in denen sich eine kationische Gruppe ableitet von mindestens einem der folgenden Monomere:

(i) Monomeren mit quartären Ammoniumgruppen der allgemeinen Formel (Mono1),

$$R^1\text{-}CH{=}CR^2\text{-}CO\text{-}Z\text{-}(C_nH_{2n})\text{-}N^{(+)}R^2R^3R^4\ A^{(-)} \qquad \text{(Mono1)}$$

in der $R^1$ und $R^2$ unabhängig voneinander stehen für Wasserstoff oder eine Methylgruppe und $R^3$, $R^4$ und $R^5$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und $A^{(-)}$ das Anion einer organischen oder anorganischen Säure ist,

(ii) Monomeren mit quartären Ammoniumgruppen der allgemeinen Formel (Mono2),

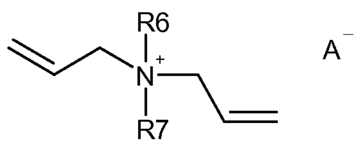

(Mono2)

worin $R^6$ und $R^7$ unabhängig voneinander stehen für eine ($C_1$ bis $C_4$)-Alkylgruppe, insbesondere für eine Methylgruppe und

$A^-$ das Anion einer organischen oder anorganischen Säure ist,

(iii) monomeren Carbonsäuren der allgemeinen Formel (Mono3),

$$R^8\text{-}CH{=}CR^9\text{-}COOH \qquad \text{(Mono3)}$$

in denen $R^8$ und $R^9$ unabhängig voneinander Wasserstoff oder Methylgruppen sind. Besonders bevorzugt sind solche Polymerisate, bei denen Monomere des Typs (i) eingesetzt werden, bei denen $R^3$, $R^4$ und $R^5$ Methylgruppen sind, Z eine NH-Gruppe und $A^{(-)}$ ein Halogenid-, Methoxysulfat- oder Ethoxysulfat-Ion ist; Acrylamidopropyl-trimethylammoniumchlorid ist ein besonders bevorzugtes Monomeres (i). Als Monomeres (ii) für die genannten Polymerisate

wird bevorzugt Acrylsäure verwendet.
Besonders bevorzugte amphotere Polymere sind Copolymere, aus mindestens einem Monomer (Mono1) bzw. (Mono2) mit dem Momomer (Mono3), insbesondere Copolymere aus den Monomeren (Mono2) und (Mono3). Erfindungsgemäß ganz besonders bevorzugt verwendete amphotere Polymere sind Copolymerisate aus Diallyl-dimethylammoniumchlorid und Acrylsäure. Diese Copolymerisate werden unter der INCI-Bezeichnung Polyquaternium-22 unter anderem mit dem Handelsnamen Merquat® 280 (Nalco) vertrieben.
Darüber hinaus können die erfindungsgemäßen amphoteren Polymere neben einem Monomer (Mono1) oder (Mono2) und einem Monomer (Mono3) zusätzlich ein Monomer (Mono4)
(iv) monomere Carbonsäureamide der allgemeinen Formel (Mono4),

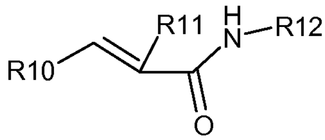

in denen $R^{10}$ und $R^{11}$ unabhängig voneinander Wasserstoff oder Methylgruppen sind und $R^{12}$ für ein Wasserstoffatom oder eine ($C_1$- bis $C_8$)-Alkylgruppe steht, enthalten.

Erfindungsgemäß ganz besonders bevorzugt verwendete amphotere Polymere auf Basis eines Comonomers (Mono4) sind Terpolymere aus Diallyldimethylammoniumchlorid, Acrylamid und Acrylsäure. Diese Copolymerisate werden unter der INCI-Bezeichnung Polyquaternium-39 unter anderem mit dem Handelsnamen Merquat® Plus 3330 (Nalco) vertrieben.

Die amphoteren Polymere können generell sowohl direkt als auch in Salzform, die durch Neutralisation der Polymerisate, beispielsweise mit einem Alkalihydroxid, erhalten wird, erfindungsgemäß eingesetzt werden.

Die zuvor genannten kationischen Polymere können einzeln oder in beliebigen Kombinationen miteinander verwendet werden, wobei Mengen zwischen 0,01 bis 10 Gew.%, bevorzugt, Mengen von 0,01 bis 7,5 Gew.% und ganz besonders bevorzugt in Mengen von 0,1 bis 5,0 Gew.% enthalten sind. Die allerbesten Ergebnisse werden dabei mit Mengen von 0,1 bis 3,0 Gew.% jeweils bezogen auf die Gesamtzusammensetzung des jeweiligen Mittels erhalten.

Ein zweiter Gegenstand der vorliegenden Erfindung sind kosmetische Zusammensetzungen enthaltend in einem kosmetischen Träger - jeweils bezogen auf das Gewicht der gesamten Zusammensetzung des Mittels

a) mindestens einen Antischuppenwirkstoff in einer Gesamtmenge von 0,01 bis 10,0 Gew.%, ausgewählt aus

i. Zink-Pyrithion,
ii. Climbazol,
iii. Octopirox,
iv. Ketoconazol,
v. Selendisulfid,
vi. selenhaltige Pflanzenöle und
vii. selenhaltige Pflanzenextrakte sowie deren Mischungen und

b) 0,01 bis 5,0 Gew.% mindestens eines Silikones enthaltend Zuckerstrukturen der folgenden Formel,

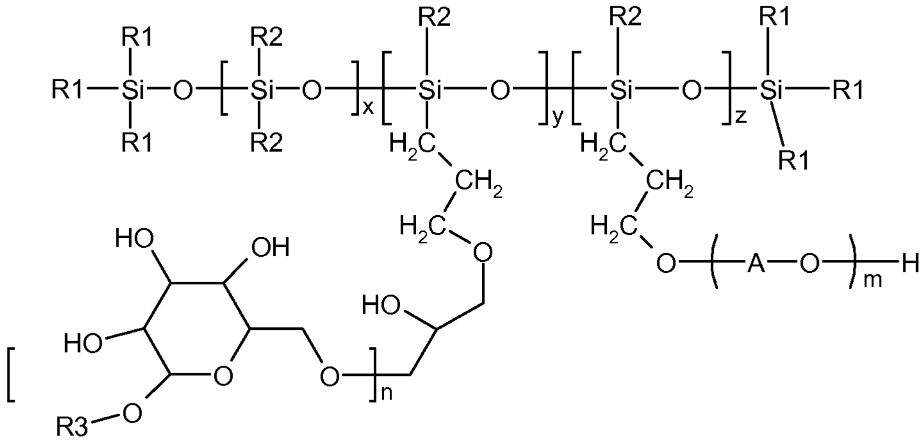

in welcher die Reste R1, R2 und R3 unabhängig voneinander stehen für eine Methyl, Ethyl, Propyl, iso-Propyl, Hydroxy, Methoxy oder Ethoxygruppe, x, y und z stehen für jeweils für eine ganze Zahl von 1 bis 1000, n und m stehen unabhängig voneinander jeweils für eine ganze Zahl von 1 bis 100, in einer Gesamtmenge von 0,01 bis 5,0 Gew.% und

c)mindestens eine quartäre Ammoniumverbindung in einer Gesamtmenge von 0,1 bis 10,0 Gew.% bezogen auf das Gewicht der gesamten Zusammensetzung, wobei die quatäre Ammoniumverbindung ausgewählt ist aus mindestens einer der Gruppen

i. der Esterquats und/oder
ii. der quarternären Imidazoline der Formel (Tkat2),

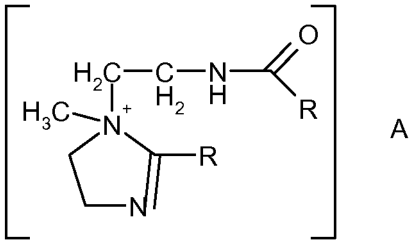

in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
iii. der Amine und/oder kationisierten Amine und/oder
iv. Poly(methacryloyloxyethyltrimethylammoniumverbindungen) und/oder;
v. quaternisierten Cellulose-Derivaten, insbesondere Polyquaternium 10, Polyquaternium-24, Polyquaternium-27, Polyquaternium-67, Polyquaternium-72, und/oder
vi. kationischen Alkylpolyglycosiden und/oder
vii. kationisertem Honig und/oder
viii. kationischen Guar-Derivaten und/oder
ix. Chitosan und/oder
x. polymeren Dimethyldiallylammoniumsalzen und deren Copolymeren mit Estern und Amiden von Acrylsäure und Methacrylsäure, insbesondere Polyquaternium-7 und/oder
xi. Copolymeren des Vinylpyrrolidons mit quaternierten Derivaten des Dialkylaminoalkylacrylats und -methacrylats, insbesondere Polyquaternium-11 und/oder
xii. Vinylpyrrolidon-Vinylimidazoliummethochlorid-Copolymeren, insbesondere Polyquaternium-16 und/oder
xiii. quaterniertem Polyvinylalkohol und/oder
xiv. Polyquaternium-74,

sowie deren Mischungen.

Bevorzugte Inhaltsstoffe der Gruppe c) sind Stearamidopropyldimethylamine und/oder Distearoylethyl Hydroxyethylmonium Methosulfate und/oder Dicocoyl Hydroxyethylmonium Methosulfate und/oder Dipalmitoylethyl Dimoniumchloride und/oder Quaternium-27 und/oder Quaternium-91 und/oder Behenoyl PG -Trimoniumchloride. Höchst bevorzugt ist es weiterhin mindestens zwei, noch bevorzugter mindestens drei Inhaltsstoffe dieser Gruppe zu wählen. Bei diesen Mischungen ist es höchst bevorzugt, wenn eine der zwei oder drei Verbindungen Stearamidopropyldimethylamin ist. Allerhöchst bevorzugt ist es, wenn weiterhin als optionale kationische Ammoniumverbindung Cetyltrimethylammoniumchlorid oder Behenyltrimethylammoniumchlorid hinzugefügt wird. Die besten Ergebnisse werden jedoch erzielt, wenn insbesondere zu den letzt genannten Zusammensetzungen enthaltend: Stearamidopropyldimethylamin und Distearoylethyl Hydroxyethylmonium Methosulfate und/oder Dicocoyl Hydroxyethylmonium Methosulfate und/oder Dipalmitoylethyl Dimoniumchloride und/oder Quaternium-27 und/oder Quaternium-91 und/oder Behenoyl PG -Trimoniumchloride und Cetyltrimethylammoniumchlorid oder Behenyltrimethylammoniumchlorid weiterhin eines der zuvor genannten kationischen Polymere, insbesondere Poly(methacryloyloxyethyltrimethylammonium-verbindungen) und/oder; quaternisierten Cellulose-Derivaten, insbesondere Polyquaternium 10, Polyquaternium-24, Polyquaternium-27, Polyquaternium-67, Polyquaternium-72, und/oder Polyquaternium-74 enthalten ist. Hierbei sind Polyquaternium-67, Polyquaternium-72 und Polyquaternium-74 am bevorzugtesten.

Erfindungsgemäß besonders bevorzugt sind diejenigen Zusammensetzungen, welche enthalten:

a) Zink-Pyrithion, Climbazol, Octopirox, Ketoconazol, Selendisulfid, selenhaltige Pflanzenöle und selenhaltige Pflanzenextrakte sowie deren Mischungen,
b) Ein Aminosilikon wie zuvor ebenfalls unter b) beschrieben und
c) Stearamidopropyldimethylamin und Distearoylethyl Hydroxyethylmonium Methosulfate und/oder Dicocoyl Hydroxyethylmonium Methosulfate und/oder Dipalmitoylethyl Dimoniumchloride und Quaternium-27 und/oder Quaternium-91 und/oder Behenoyl PG -Trimoniumchloride und Cetyltrimethylammoniumchlorid oder Behenyltrimethylammoniumchlorid und weiterhin Poly(methacryloyloxyethyltrimethyl-ammoniumverbindungen) und/oder Polyquaternium-67 und/oder Polyquaternium-72 und/oder Polyquaternium-74.

Weiterhin ist es erfindungsgemäß höchst bevorzugt, wenn in den erfindungsgemäßen Zusammensetzungen mindestens ein amphoteres und/oder zwitterionisches Tensid enthalten ist. In den erfindungsgemäßen Zusammensetzungen tragen diese Inhaltsstoffe möglicherweise erheblich zur Verbesserung der Haftung der Antischuppenwirkstoffe auf der keratinischen Faser bei.

Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden (Tampho) werden solche oberflächenaktiven Verbindungen verstanden, die zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Besonders bevorzugte ampholytische Tenside sind das N-Kokosalkylaminopropionat, das Kokosacylaminoethylaminopropionat und das $C_{12}$ - $C_{18}$ - Acylsarcosin. Eine besonders bevorzugte Verbindung ist das Coco Betaine.

Diese Inhaltsstoffe werden in Mengen von 0,01 bis 8,0 Gew.% in Bezug auf die gesamte Zusammensetzung des Mittels verwendet. Mengen von 0,05 bis 7,0 Gew.% sind dabei bevorzugt. Besonders bevorzugt sind Mengen von 0,1 bis 6,0 Gew.%, höchst bevorzugt von 0,3 bis 3,0 Gew.%.

Ein dritter Gegenstand der vorliegenden Erfindung sind daher kosmetische Zusammensetzungen enthaltend in einem kosmetisch verträglichen Träger - jeweils bezogen auf das Gewicht der gesamten Zusammensetzung -

a) mindestens einen Antischuppenwirkstoff in einer Gesamtmenge von 0,01 bis 10,0 Gew.%, ausgewählt aus

i. Zink-Pyrithion,
ii. Climbazol,
iii. Octopirox,
iv. Ketoconazol,
v. Selendisulfid,
vi. selenhaltige Pflanzenöle und
vii. selenhaltige Pflanzenextrakte sowie deren Mischungen und

b) 0,01 bis 5,0 Gew.% mindestens eines Silikones enthaltend Zuckerstrukturen der folgenden Formel,

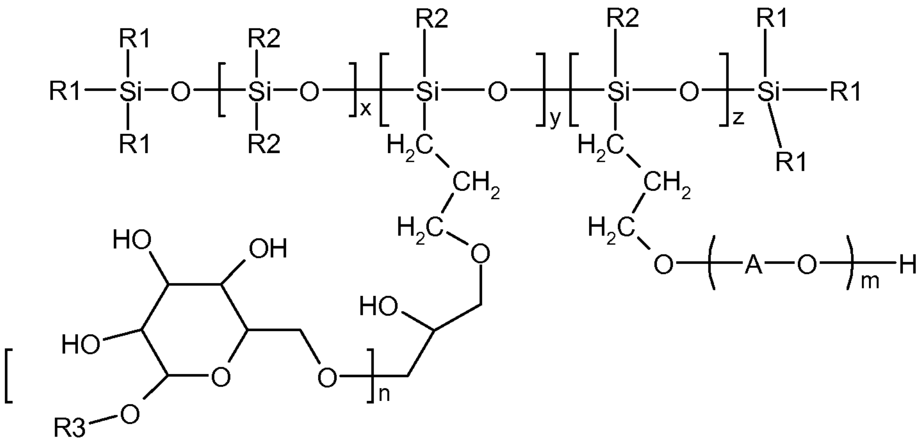

in welcher die Reste R1, R2 und R3 unabhängig voneinander stehen für eine Methyl, Ethyl, Propyl, iso-Propyl, Hydroxy, Methoxy oder Ethoxygruppe, x, y und z stehen für jeweils für eine ganze Zahl von 1 bis 1000, n und m stehen unabhängig voneinander jeweils für eine ganze Zahl von 1 bis 100, in einer Gesamtmenge von 0,01 bis 5,0 Gew.% und

c) mindestens eine quartäre Ammoniumverbindung in einer Gesamtmenge von 0,1 bis 10,0 Gew.% bezogen auf das Gewicht der gesamten Zusammensetzung, ausgewählt aus mindestens einer der Gruppen

i. der Esterquats und/oder

ii. der quarternären Imidazoline der Formel (Tkat2),

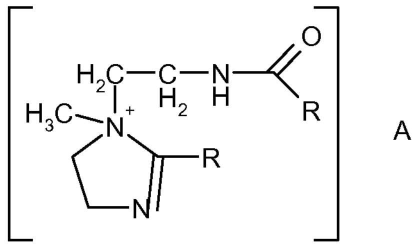

in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder

iii. der Amine und/oder kationisierten Amine und/oder

iv. Poly(methacryloyloxyethyltrimethylammoniumverbindungen) und/oder;

v. quaternisierten Cellulose-Derivaten, insbesondere Polyquaternium 10, Polyquaternium-24, Polyquaternium-27, Polyquaternium-67, Polyquaternium-72, und/oder

vi. kationischen Alkylpolyglycosiden und/oder

vii. kationisertem Honig und/oder

viii. kationischen Guar-Derivaten und/oder

ix. Chitosan und/oder

x. polymeren Dimethyldiallylammoniumsalzen und deren Copolymeren mit Estern und Amiden von Acrylsäure und Methacrylsäure, insbesondere Polyquaternium-7 und/oder

xi. Copolymeren des Vinylpyrrolidons mit quaternierten Derivaten des Dialkylaminoalkylacrylats und -methacrylats, insbesondere Polyquaternium-11 und/oder

xii. Vinylpyrrolidon-Vinylimidazoliummethochlorid-Copolymeren, insbesondere Polyquaternium-16 und/oder

xiii. quaterniertem Polyvinylalkohol und/oder

xiv. Polyquaternium-74 und

d) 0,01 Gew.% bis 8,0 Gew.% mindestens eines Tensides ausgewählt aus den zwitterionischen und/oder amphoteren Tensiden.

Besonders bevorzugte Zusammensetzungen dieser Ausführungsform enthalten als Inhaltsstoffe der Gruppe c) Stearamidopropyldimethylamine und/oder Distearoylethyl Hydroxyethylmonium Methosulfate und/oder Dicocoyl Hydroxyethylmonium Methosulfate und/oder Dipalmitoylethyl Dimoniumchloride und/oder Quaternium-27 und/oder Quaternium-91 und/oder Behenoyl PG - Trimoniumchloride. Höchst bevorzugt ist es weiterhin mindestens zwei, noch bevorzugter mindestens drei Inhaltsstoffe dieser Gruppe zu wählen. Bei diesen Mischungen ist es höchst bevorzugt, wenn eine der zwei oder drei Verbindungen Stearamidopropyldimethylamin ist. Allerhöchst bevorzugt ist es, wenn weiterhin als optionale kationische Ammoniumverbindung Cetyltrimethylammoniumchlorid oder Behenyltrimethylammoniumchlorid hinzugefügt wird. Die besten Ergebnisse werden jedoch erzielt, wenn insbesondere zu den letzt genannten Zusammensetzungen enthaltend: Stearamidopropyldimethylamin und Distearoylethyl Hydroxyethylmonium Methosulfate und/oder Dicocoyl Hydroxyethylmonium Methosulfate und/oder Dipalmitoylethyl Dimoniumchloride und/oder Quaternium-27 und/oder Quaternium-91 und/oder Behenoyl PG -Trimoniumchloride und Cetyltrimethylammoniumchlorid oder Behenyltrimethylammoniumchlorid weiterhin eines der zuvor ebenfalls genannten kationischen Polymere, insbesondere Poly(methacryloyloxyethyltrimethylammoniumverbindungen) und/oder; quaternisierten Cellulose-Derivaten, insbesondere Polyquaternium 10, Polyquaternium-24, Polyquaternium-27, Polyquaternium-67, Polyquaternium-72, und/oder Polyquaternium-74 enthalten ist. Hierbei sind Polyquaternium-67, Polyquaternium-72 und Polyquaternium-74 am bevorzugtesten.

Erfingungsgemäß besonders bevorzugt sind diejenigen Zusammensetzungen, welche enthalten:

a) Zink-Pyrithion, Climbazol, Octopirox, Ketoconazol, Selendisulfid, selenhaltige Pflanzenöle und selenhaltige Pflanzenextrakte sowie deren Mischungen,

b) ein Silikones enthaltend Zuckerstrukturen wie bei den Ausführungsformen zuvor ebenfalls unter b) beschrieben und

c) Stearamidopropyldimethylamin und Distearoylethyl Hydroxyethylmonium Methosulfate und/oder Dicocoyl Hydroxyethylmonium Methosulfate und/oder Dipalmitoylethyl Dimoniumchloride und Quaternium-27 und/oder Quaternium-91 und/oder Behenoyl PG -Trimoniumchloride und Cetyltrimethylammoniumchlorid oder Behenyltrimethylammoniumchlorid und weiterhin Poly(methacryloyloxyethyltrimethyl-ammoniumverbindungen) und/oder Polyquaternium-67 und/oder Polyquaternium-72 und/oder Polyquaternium-74 und

d) Cocamidopropylbetain und/oder Coco Betaine.

Zu diesem höchst bevorzgten Grundgerüst an Inhaltsstoffen können weiterhin alle in kosmetischen Zusammensetzungen üblichen Inhaltsstoffe zugegeben werden. Die Auswahl dieser Bestandteile richtet sich im Allgemeinen nach der beabsichtigten Verwendung der Haarbehandlungsmittel.

Neben den zuvor beschriebenen zwingenden Silikonen können die erfindungsgemäßen Zusammensetzungen weitere Silikone enthalten. Diese optionalen Silikone sind bevorzugt mindestens ein Silikonpolymer ausgewählt aus der Gruppe der Dimethiconole und/oder der Gruppe der aminofunktionellen Silikone und/oder der Gruppe der Dimethicone und / oder der Gruppe der Cyclomethicone.

Die erfindungsgemäßen Dimethicone können sowohl linear als auch verzweigt als auch cyclisch oder cyclisch und verzweigt sein. Lineare Dimethicone können durch die folgende Strukturformel (Si1) dargestellt werden:

$$(SiR^1_3)\text{-}O\text{-}(SiR^2_2\text{-}O\text{-})_x\text{-}(SiR^1_3) \qquad (Si1)$$

Verzweigte Dimethicone können durch die Strukturformel (Si1.1) dargestellt werden:

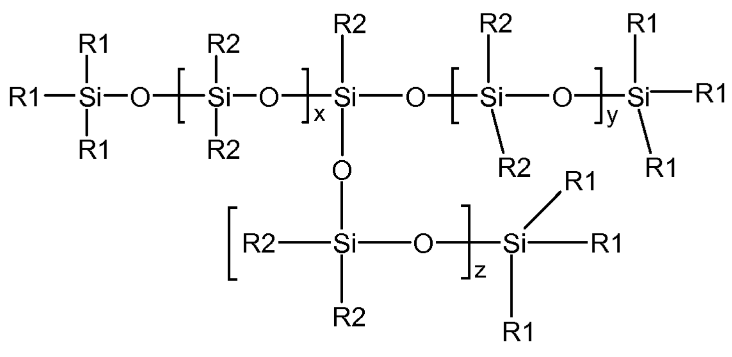

(Si1.1)

Die Reste $R^1$ und $R^2$ stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Die Zahlen

x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs. Höchst bevorzugt sind Viskositäten um den Bereich von etwa 60.000 cPs herum. Beispielhaft sei hier auf das Produkt "Dow Corning 200 mit 60000cSt" verwiesen.

Besonders bevorzugte erfindungsgemäße kosmetische oder dermatologische Zubereitungen sind dadurch gekennzeichnet, dass sie mindestens ein Silikon der Formel (Si1.2)

$$(CH_3)_3Si\text{-}[O\text{-}Si(CH_3)_2]_x\text{-}O\text{-}Si(CH_3)_3 \qquad (Si1.2),$$

enthalten, in der x für eine Zahl von 0 bis 100, vorzugsweise von 0 bis 50, weiter bevorzugt von 0 bis 20 und insbesondere 0 bis 10, steht.

Die Dimethicone (Si1) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% bezogen auf die gesamte Zusammensetzung enthalten. Schließlich werden unter den Silikonverbindungen die Dimethiconole (Si8) verstanden. Die erfindungsgemäßen Dimethiconole können sowohl linear als auch verzweigt als auch cyclisch oder cyclisch und verzweigt sein. Lineare Dimethiconole können durch die folgende Strukturformel (Si8 - I) dargestellt werden:

$$(SiOHR^1_2)\text{-}O\text{-}(SiR^2_2\text{-}O\text{-})_x\text{-}(SiOHR^1_2) \qquad (Si8 - I)$$

Verzweigte Dimethiconole können durch die Strukturformel (Si8 - II) dargestellt werden:

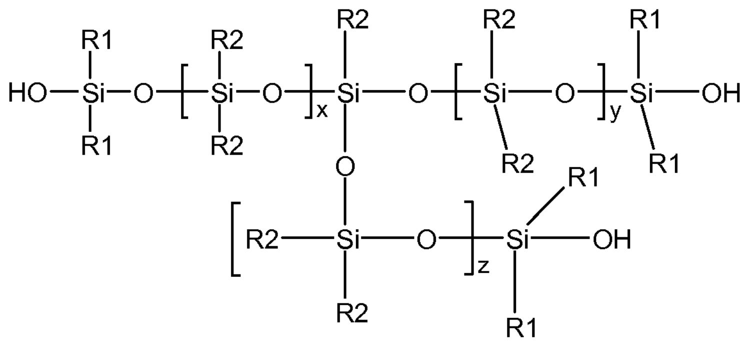

(Si8-II)

Die Reste $R^1$ und $R^2$ stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Die Zahlen x, y und z sind ganze Zahlen und laufen jeweils unabhängig voneinander von 0 bis 50.000. Die Molgewichte der Dimethiconole liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Als Beispiele für derartige Produkte werden die folgenden Handelsprodukte genannt: Dow Corning 1-1254 Fluid, Dow Corning 2-9023 Fluid, Dow Corning 2-9026 Fluid, Abil OSW 5 (Degussa Care Specialties), Dow Corning 1401 Fluid, Dow Corning 1403 Fluid, Dow Corning 1501 Fluid, Dow Corning 1784 HVF Emulsion, Dow Corning 9546 Silicone Elastomer Blend, SM555, SM2725, SM2765, SM2785 (alle vier zuvor genannten GE Silicones), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH). Die Dimethiconole (Si8) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconol bezogen auf die Zusammensetzung.

Besonders bevorzugte erfindungsgemäße Mittel enthalten ein oder mehrere aminofunktionelle Silikone. Solche Silikone können z.B. durch die Formel (Si-2)

$$M(R_aQ_bSiO_{(4-a-b)/2})_xR_cSiO_{(4-c)/2})_yM \qquad (Si-2)$$

Beschrieben werden, wobei in der obigen Formel

- R ein Kohlenwasserstoff oder ein Kohlenwasserstoffrest mit 1 bis etwa 6 Kohlenstoffatomen ist,
- Q ein polarer Rest der allgemeinen Formel $-R^1HZ$ ist, worin
  $R^1$ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und
  Z ein organischer, aminofunktioneller Rest ist, der mindestens eine aminofunktionelle Gruppe enthält;
- a Werte im Bereich von etwa 0 bis etwa 2 annimmt,
- b Werte im Bereich von etwa 1 bis etwa 3 annimmt,
- a + b kleiner als oder gleich 3 ist, und
- c eine Zahl im Bereich von etwa 1 bis etwa 3 ist, und
- x eine Zahl im Bereich von 1 bis etwa 2.000, vorzugsweise von etwa 3 bis etwa 50 und am bevorzugtesten von etwa 3 bis etwa 25 ist, und
- y eine Zahl im Bereich von etwa 20 bis etwa 10.000, vorzugsweise von etwa 125 bis etwa 10.000 und am bevorzugtesten von etwa 150 bis etwa 1.000 ist, und
- M eine geeignete Silikon-Endgruppe ist, wie sie im Stande der Technik bekannt ist, vorzugsweise Trimethylsiloxy.

Z ist gemäß Formel (Si-2) ein organischer, aminofunktioneller Rest, enthaltend mindestens eine funktionelle Aminogruppe. Eine mögliche Formel für besagtes Z ist $NH(CH_2)_zNH_2$, worin z eine ganze Zahl von größer gleich 1 ist. Eine andere mögliche Formel für besagtes Z ist $-NH(CH_2)_z(CH_2)_{zz}NH$, worin sowohl z als auch zz unabhängig voneinander eine ganze Zahl von größer gleich 1 sind, wobei diese Struktur Diamino-Ringstrukturen umfasst, wie Piperazinyl. Besagtes Z ist am bevorzugtesten ein $-NHCH_2CH_2NH_2$-Rest. Eine andere mögliche Formel für besagtes Z ist-$N(CH_2)_z(CH_2)_{zz}NX_2$ oder $-NX_2$, worin jedes X von $X_2$ unabhängig ausgewählt ist aus der Gruppe bestehend aus Wasserstoff und Alkylgruppen mit 1 bis 12 Kohlenstoffatomen, und zz 0 ist.

Q gemäß Formel (Si-2) ist am bevorzugtesten ein polarer aminofunktioneller Rest der Formel -$CH_2CH_2CH_2NHCH_2CH_2NH_2$.

In der Formel (Si-2) nimmt a Werte im Bereich von 0 bis 2 an, b nimmt Werte im Bereich von 2 bis 3 an, a + b ist kleiner als oder gleich 3, und c ist eine Zahl im Bereich von 1 bis 3. Erfindungsgemäß geeignet sind kationische Silikonöle wie beispielsweise die im Handel erhältlichen Produkte Dow Corning (DC) 929 Emulsion, DC 2-2078, DC 5-7113, SM-2059 (General Electric) sowie SLM-55067 (Wacker).

Besonders bevorzugte erfindungsgemäße Mittel sind dadurch gekennzeichnet, dass sie mindestens es ein aminofunktionelles Silikon der Formel (Si3-a)

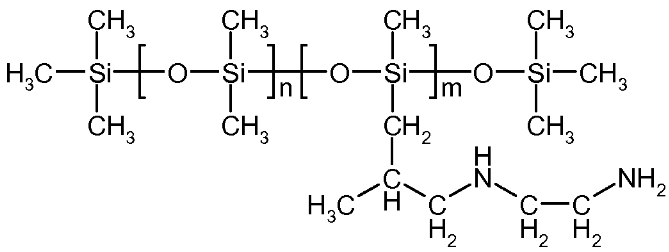

(Si-3a),

enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet und sind beispielsweise unter der Bezeichnung Q2-7224 (Hersteller: Dow Corning; ein stabilisiertes Trimethylsilylamodimethicon) erhältlich.

Besonders bevorzugt sind auch erfindungsgemäße Mittel, die mindestens ein aminofunktionelles Silikon der Formel (Si-3b)

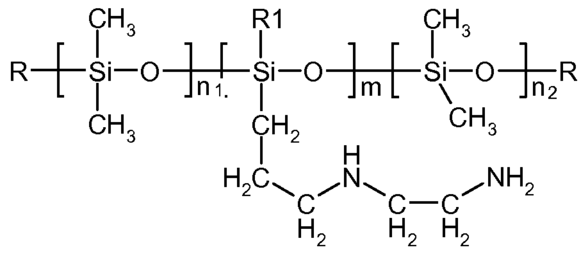

(Si-3b),

enthalten, worin

| | |
|---|---|
| R | für -OH, eine (gegebenenfalls ethoxylierte und/oder propoxylierte) ($C_1$ bis $C_{20}$)-Alkoxygruppe oder eine $-CH_3$-Gruppe steht, |
| R1 | für -OH, eine ($C_1$ bis $C_{20}$)-Alkoxygruppe oder eine $-CH_3$-Gruppe und |
| m, n1 und n2 | Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt. |

Diese Silikone werden nach der INCI-Deklaration als Amodimethicone, bzw. als funktionalisierte Amodimethicone, wie beispielsweise Bis(C13-15 Alkoxy) PG Amodimethicone (beispielsweise als Handelsprodukt: DC 8500 der Firma Dow Corning erhältlich), Trideceth-9 PG-Amodimethicone (beispielsweise als Handelsprodukt Silcare Silicone SEA der Firma Clariant erhältlich) bezeichnet. Geeignete diquaternäre Silikone sind ausgewählt aus Verbindungen der allgemeinen Formel (Si3c)

$$[R^1R^2R^3N^+\text{-A-SiR}^7R^8\text{-(O-SiR}^9R^{10})_n\text{-O-SiR}^{11}R^{12}\text{-A-N+}R^4R^5R^6]\ 2X^- \qquad \text{(Si3c)}$$

wobei die Reste R1 bis R6 unabhängig voneinander C1-bis C22-Alkylreste bedeuten, welche Hydroxygruppen enthalten können und wobei vorzugsweise mindestens einer der Reste mindestens 8 C-Atome aufweist und die übrigen Reste 1 bis 4 C-Atome aufweisen,
die Reste R7 bis R12 unabhängig voneinander gleich oder verschieden sind und C1-bis C10-Alkyl oder Phenyl bedeuten,
A eine divalente organische Verbindungsgruppe bedeutet,
n eine Zahl von 0 bis 200, vorzugsweise von 10 bis 120, besonders bevorzugt von 10 bis 40 ist, und $X^-$ ein Anion ist.

Die divalente Verbindungsgruppe ist vorzugsweise eine C1-bis C12-Alkylen-oder Alkoxyalkylengruppe, die mit einer oder mehreren Hydroxylgruppen substituiert sein kann. Besonders bevorzugt ist die Gruppe $-(CH_2)_3-O-CH_2-CH(OH)-CH_2-$.

Das Anion $X^-$ kann ein Halogenidion, ein Acetat, ein organisches Carboxylat oder eine Verbindung der allgemeinen Formel $RSO_3^-$ sein, worin R die Bedeutung von C1-bis C4-Alkylresten hat.

Ein bevorzugtes diquaternäres Silikon hat die allgemeine Formel (Si3d)

$$[RN^+Me_2\text{-A-(SiMe}_2O)_n\text{-SiMe}_2\text{-A-N}^+Me_2R]2CH_3COO^- \qquad \text{(Si3d)},$$

wobei A die Gruppe $-(CH_2)_3-O-CH_2-CH(OH)-CH_2-$ist,
R ein Alkylrest mit mindestens 8 C-Atomen und n eine Zahl von 10 bis 120 ist.

Geeignete Silikonpolymere mit zwei endständigen, quaternären Ammoniumgruppen sind unter der INCI-Bezeichnung Quaternium-80 bekannt. Hierbei handelt es sich um Dimethylsiloxane mit zwei endständigen Trialkylammoniumgruppen. Derartige diquaternäre Polydimethylsiloxane werden von der Firma Evonik unter den Handelsnamen Abil® Quat 3270, 3272 und 3474 vertrieben. Erfindungsgemäß bevorzugte Haarbehandlungsmittel sind dadurch gekennzeichnet, dass sie, bezogen auf ihr Gewicht, 0,01 bis 10 Gew.%, vorzugsweise 0,01 bis 8 Gew.%, besonders bevorzugt 0,1 bis 7,5 Gew.% und insbesondere 0,2 bis 5 Gew.% aminofunktionelle(s) Silikon(e) und/oder diquaternäres Silikon enthalten.

Weitere kationische Silikonverbindungen mit mindestens drei endständigen aminofunktionalen Gruppen können erfindungsgemäß ebenfalls verwendet werden. Diese kationischen Silikonpolymere zeichnen sich dadurch aus, dass sie ein Silikongerüst sowie gegebenenfalls einen Polyetherteil und weiterhin mindestens einen Teil mit Ammoniumstruktur aufweisen. Beispiele für die bevorzugten kationischen Silikonpolymere im Sinne der vorliegenden Erfindung sind insbesondere die Verbindungen mit den INCI - Bezeichnungen: Silicone Quaternium-1, Silicone Quaternium-2, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, Silicone Quaternium-16, Silicone Quaternium-17, Silicone Quaternium-18, Silicone Quaternium-20, Silicone Quaternium-21, Silicone Quaternium-22 sowie Silicone Quaternium-2 Panthenol Succinate und Silicone Quaternium-16/Glycidyl Dimethicone Crosspolymer. Am bevorzugtesten ist insbesondere Silicone Quaternium-22. Dieser Rohstoff wird beispielsweise von der Firma Evonik unter der Handelsbezeichnung Abil® T-Quat 60 vertrieben.

Ein weiteres bevorzugtes kationisches Aminosilikon entspricht der folgenden Formel:

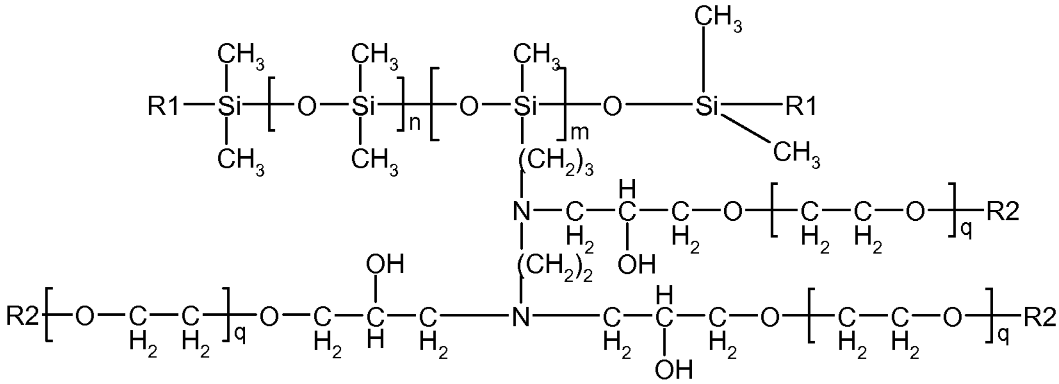

in welcher R1 steht für eine Methyl-, Ethyl-, Hydroxy-, Methoxy- oder Ethoxygruppe,
R2, steht für einen geradkettigen oder verzweigten C8 bis C24 Alkyl- oder Alkylenrest, vorzugsweise einen geradkettigen oder verzweigten C9 bis C22 Alkyl- oder Alkenylrest, besonders bevorzugt einen geradkettigen oder verzweigten C11 bis C18 Alkyl- oder Alkenylrest, höchst bevorzugt einen entsprechenden Alkylrest,
n und m jeweils für ganze Zahlen von 1 bis 1000 stehen und q steht jeweils für eine ganze Zahl von 2 bis 50, bevorzugt 4 bis 30, besonders bevorzugt 4 bis 18 und höchst bevorzugt von 4 bis 12.

Das Molekulargewicht derartiger Verbindungen beträgt 15.000 bis 2.000.000, gemessen mit einem Brookfield Rotationsviskosimeter RV, Spindel 5, bei 20 °C. Bevorzugt beträgt das Molgewicht 30.000 bis 1.750.000 und besonders bevorzugt 50.000 bis 1.500.000. Der Stickstoffgehalt der erfindungsgemäßen Silikone beträgt 0,03 bis 4,2 Gew.%, bevorzugt 0,1 bis 2,8 Gew.% und höchst bevorzugt 0,16 bis 1,4 Gew.%. Erfindungsgemäße aminofunktionelle kationische Silikone der obigen Formel können beispielsweise von der Fa. Clariant bezogen werden. Ein erfindungsgemäß höchst bevorzugtes Produkt ist unter der INCI-Bezeichnung Trideceth-9-Amodimethicone and Trideceth-12 im Handel erhältlich.

Ein weiteres besonders bevorzugtes aminofunktionales Silikon ist mindestens ein 4-morpholinomethyl-substituiertes Silikon der Formel (V),

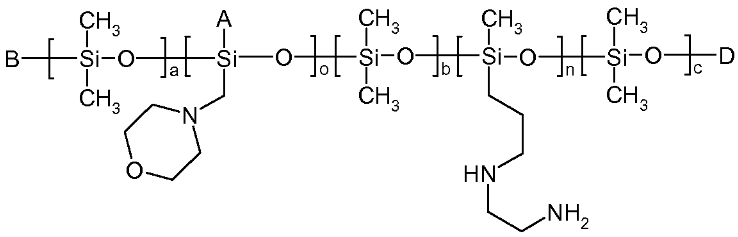

(V),

in der

A für eine über ein -O- gebundene Struktureinheit (I), (II) oder (III)

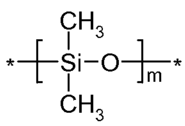

(I)

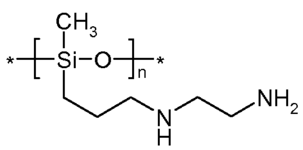

(II)

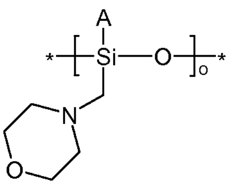

(III),

oder einen über ein -O- gebundenen oligomeren oder polymeren Rest enthaltend Struktureinheiten der Formeln (I), (II) oder (III) oder die Hälfte eines verbindenden O-Atoms zu einer Struktureinheit (III) oder für -OH steht,

| | |
|---|---|
| * | für eine Bindung zu einer der Struktureinheiten (I), (II) oder (III) oder für eine Endgruppe B (Si-gebunden) oder D (O-gebunden) steht, |
| B | für eine Gruppe -OH, $-O-Si(CH_3)_3$,$-O-Si(CH_3)_2OH$ ,$-O-Si(CH_3)_2OCH_3$ steht, |
| D | für eine Gruppe -H, $-Si(CH_3)_3$,$-Si(CH_3)_2OH$, $-Si(CH_3)_2OCH_3$ steht, |
| a, b und c | für ganze Zahlen zwischen 0 und 1000 stehen, mit der Maßgabe $a + b + c > 0$ |
| m, n und o | für ganze Zahlen zwischen 1 und 1000 stehen. |

Derartige aminofunktionale Silikone tragen die INCI - Bezeichnung Amodimethicone/Morpholinomethyl Silsesquioxane Copolymer. Ein besonders geeignetes Amodimethicone ist das Produkt mit der Handelsbezeichnung Wacker Belsil® ADM 8301E. Erfindungsgemäß kann es besonders vorteilhaft sein, wenn als Silikone ausschließlich die zuvor genannten Silikone verwendet werden.

Diese letztgenannten kationischen aminofunktionellen Silikonpolymere sind in den erfindungsgemäßen Zusammensetzungen in Mengen von 0,01 bis 5 Gew.%, bevorzugt in Mengen von 0,05 bis 5 Gew.% und ganz besonders bevorzugt in Mengen von 0,1 bis 5 Gew.% enthalten. Die allerbesten Ergebnisse werden dabei mit Mengen von 0,1 bis 2,5 Gew.% jeweils bezogen auf die Gesamtzusammensetzung des jeweiligen Mittels erhalten.

Eine weitere erfindungsgemäße Silikonverbindung mit Aminofunktionen sind Polyammonium-Polysiloxan Verbindungen. Die Polyammonium-Polysiloxan Verbindungen können beispielsweise unter der Handelsbezeichnung Baysilone® von GE Bayer Silicones bezogen werden. Die Produkte mit den Bezeichnungen Baysilone TP 3911, SME 253 und SFE 839 sind dabei bevorzugt. Ganz besonders bevorzugt ist die Verwendung von Baysilone TP 3911 als Wirkkomponente der erfindungsgemäßen Zusammensetzungen. Die Polyammonium-Polysiloxan Verbindungen werden in dem erfindungsgemäßen Zusammensetzungen in einer Menge von 0,01 bis 10 Gew.% , vorzugsweise 0,01 bis 7,5, besonders bevorzugt 0,01 bis 5,0 Gew.%, ganz besonders bevorzugt von 0,05 bis 2,5 Gew.% jeweils in Bezug auf die Gesamtzusammensetzung verwendet.

Auch die nach INCI als Cyclomethicone bezeichneten cyclischen Dimethicone sind erfindungsgemäß mit Vorzug einsetzbar. Hier sind erfindungsgemäße kosmetische oder dermatologische Zubereitungen bevorzugt, die mindestens ein Silikon der Formel (Si-4)

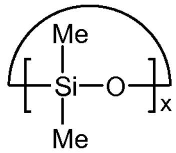

(Si-4)

enthalten, in der x für eine Zahl von 3 bis 200, vorzugsweise von 3 bis 10, weiter bevorzugt von 3 bis 7 und insbesondere 3, 4, 5 oder 6, steht.

Erfindungsgemäß ebenfalls bevorzugte Mittel sind dadurch gekennzeichnet, dass sie mindestens ein Silikon der Formel (Si-5)

$$R_3Si-[O-SiR_2]_x-(CH_2)_n-[O-SiR_2]_y-O-SiR_3 \qquad (Si-5),$$

enthalten, in der R für gleiche oder verschiedene Reste aus der Gruppe -H, -Phenyl, -Benzyl, $-CH_2-CH(CH_3)Ph$, der $C_{1-20}$-Alkylreste, vorzugsweise $-CH_3$, $-CH_2CH_3$, $-CH_2CH_2CH_3$, $-CH(CH_3)_2$, $-CH_2CH_2CH_2H_3$, $-CH_2CH(CH_3)_2$, $-CH(CH_3)CH_2CH_3$, $-C(CH_3)_3$, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Als weitere Silikone neben den erfindungsgemäßen Dimethiconen, Dimethiconolen, Amodimethiconen

und/oder Cyclomethiconen können wasserlösliche Silikone in den erfindungsgemäßen Zusammensetzungen enthalten sein.

Entsprechende hydrophile Silikone werden beispielsweise aus den Verbindungen der Formeln (Si-6) und/oder (Si-7) ausgewählt. Insbesondere bevorzugte wasserlösliche Tenside auf Silikonbasis sind ausgewählt aus der Gruppe der Dimethiconcopolyole die bevorzugt alkoxyliert, insbesondere polyethoxyliert oder polypropoxyliert sind.

Unter Dimethiconcopolyolen werden erfindungsgemäß bevorzugt Polyoxyalkylen-modifizierte Dimethylpolysiloxane der allgemeinen Formeln (Si-6) oder (Si-7) verstanden:

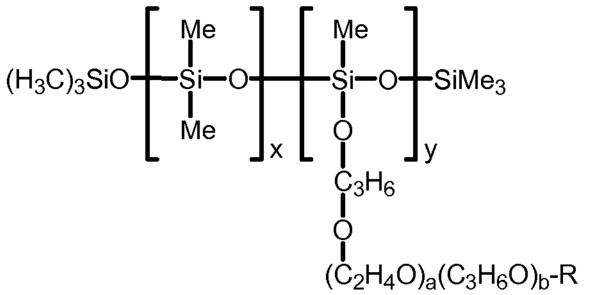

(Si-6)

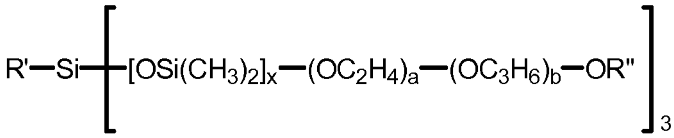

(Si-7)

worin der Rest R steht für ein Wasserstoffatom, eine Alkylgruppe mit 1 bis 12 C-Atomen, eine Alkoxygruppe mit 1 bis 12 C-Atomen oder eine Hydroxylgruppe, die Reste R' und R" bedeuten Alkylgruppen mit 1 bis 12 C-Atomen, x steht für eine ganze Zahl von 1 bis 100, bevorzugt von 20 bis 30, y steht für eine ganze Zahl von 1 bis 20, bevorzugt von 2 bis 10 und a und b stehen für ganze Zahlen von 0 bis 50, bevorzugt von 10 bis 30.

Besonders bevorzugte Dimethiconcopolyole im Sinne der Erfindung sind beispielsweise die kommerziell unter dem Handelsnamen SILWET (Union Carbide Corporation) und DOW CORNING vertriebenen Produkte. Erfindungsgemäß besonders bevorzugte Dimethiconcopolyole sind Dow Corning 190 und Dow Corning 193.

Die Dimethiconcopolyole sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconcopolyol bezogen auf die Zusammensetzung. Esteröle können mit besonderem Vorzug als Ölkörper in erfindungsgemäßen Wirkstoffkombination enthalten sein. Die Esteröle sind wie folgt definiert:

Unter Esterölen sind zu verstehen die Ester von $C_6$ - $C_{30}$ - Fettsäuren mit $C_2$ - $C_{30}$ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).

Selbstverständlich können die Esteröle auch mit Etyhlenoxid, Propylenoxid oder Mischungen aus Ethylenoxid und Propylenoxid alkoxiliert sein. Die Alkoxylierung kann dabei sowohl am Fettalkoholpart als auch am Fettsäurepart als auch an beiden Teilen der Esteröle zu finden sein. Bevorzugt ist erfindungsgemäß jedoch, wenn zunächst der Fettalkohol alkoxyliert wurde und anschließend mit Fettsäure verestert wurde. In der Formel (D4-II) sind allgemein diese Verbindungen dargestellt.

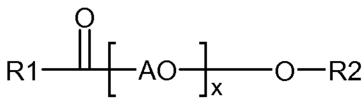

(D4-II)

R1 steht hierbei für einen gesättigten oder ungesättigten, verzweigten oder unverzweigten, cyclischen gesättigten cyclischen ungesättigten Acylrest mit 6 bis 30 Kohlenstoffatomen,
AO steht für Ethylenoxid, Propylenoxid oder Butylenoxid,
X steht für eine Zahl zwischen 1 und 200, vorzugsweise 1 und 100, besonders bevorzugt zwischen 1 und 50, ganz besonders bevorzugt zwischen 1 und 20, höchst bevorzugt zwischen 1 und 10 und am bevorzugtesten zwischen 1 und 5,
R2 steht für einen gesättigten oder ungesättigten, verzweigten oder unverzweigten, cyclischen gesättigten cyclischen ungesättigten Alkyl-, Alkenyl-, Alkinyl-, Phenyl- oder Benzylrest mit 6 bis 30 Kohlenstoffatomen. Beispiele für eingesetzte Fettsäurenanteile als Rest R1 in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile als Rest R2 in den Esterölen sind Benzylalkohol, Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Ein erfindungsgemäß besonders bevorzugtes Esteröl ist beispielsweise unter der INCI - Bezeichnung PPG-3 Benzyl Ether Myristate erhältlich.

Weiterhin sind unter Esterölen zu verstehen:

- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat, sowie
- symmetrische, unsymmetrische oder cyclische Ester der Kohlensäure mit Fettalkoholen, beispielsweise Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Esteröle werden in den erfindungsgemäßen Mitteln in einer Menge von 0,01 bis 20 Gew.%, bevorzugt 0,01 bis 10,0 Gew.%, besonders bevorzugt 0,01 bis 7,5 Gew.%, höchst bevorzugt von 0,1 bis 5,0 Gew.% verwendet. Selbstverständlich ist es erfindungsgemäß auch möglich mehrere Esteröle gleichzeitig zu verwenden.

Weiterer erfindungsgemäße Ölkörper sind:

- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.

Als natürliche Öle werden beispielsweise Amaranthsamenöl, Aprikosenkernöl, Arganöl, Avocadoöl, Babassuöl,

Baumwollsaatöl, Borretschsamenöl, Camelinaöl, Distelöl, Erdnußöl, Granatapfelkernöl, Grapefruitsamenöl, Hanföl, Haselnussöl, Holundersamenöl, Johannesbeersamenöl, Jojobaöl, Kakaobutter, Leinöl, Macadamianussöl, Maiskeimöl, Mandelöl, Marulaöl, Nachtkerzenöl, Olivenöl, Palmöl, Rapsöl, Reisöl, Sanddornfruchtfleischöl, Sanddornkernöl, Sesamöl, Sheabutter, Sojaöl, Sonnenblumenöl, Traubenkernöl, Walnußöl oder Wildrosenöl verwendet.

Selbstverständlich enthalten die erfindungsgemäßen Haarbehandlungsmittel neben der erfindungsgemäßen Wirkstoffkombination auch weitere in kosmetischen Zusammensetzungen übliche Bestandteile. Die Auswahl dieser Bestandteile richtet sich im allgemeinen nach der beabsichtigten Verwendung der Haarbehandlungsmittel. Im Falle eines Shampoos werden beispielsweise weitere oberflächenaktive Substanzen enthalten sein. Im Falle von Haarkuren werden gegebenenfalls weitere kationische Verbindungen und weitere Pflegestoffe enthalten sein. In vielen Fällen enthalten die Mittel mindestens eine oberflächenaktive Substanz, wobei prinzipiell sowohl anionische als auch zwitterionische, ampholytische, nichtionische und kationische oberflächenaktive Substanzen geeignet sind. Die Auswahl der oberflächenaktiven Substanzen richtet sich nach der Art des Mittels.

Als anionische Tenside eignen sich in erfindungsgemäßen Zubereitungen alle für die Verwendung am menschlichen Körper geeigneten anionischen oberflächenaktiven Stoffe. Typische Beispiele für anionische Tenside sind:

- lineare und verzweigte Fettsäuren mit 8 bis 30 C-Atomen (Seifen),
- Ethercarbonsäuren der Formel $R\text{-}O\text{-}(CH_2\text{-}CH_2O)_x\text{-}CH_2\text{-}COOH$, in der R eine lineare Alkylgruppe mit 8 bis 30 C-Atomen und x = 0 oder 1 bis 16 ist und deren Salze,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen.
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel $R\text{-}O(CH_2\text{-}CH_2O)_x\text{-}OSO_3H$, in der R eine bevorzugt lineare Alkylgruppe mit 8 bis 30 C-Atomen und x = 0 oder 1 bis 12 ist,
- Hydroxysulfonate im wesentlichen entsprechend mindestens einer der beiden folgenden Formeln oder deren deren Mischungen sowie deren salzen, $CH_3\text{-}(CH_2)_y\text{-}CHOH\text{-}(CH_2)_p\text{-}(CH\text{-}SO_3M)\text{-}(CH_2)_z\text{-}CH_2\text{-}O\text{-}(C_nH_{2n}O)_x\text{-}H$, und/oder $CH_3\text{-}(CH_2)_y\text{-}(CH\text{-}SO_3M)\text{-}(CH_2)_p\text{-}CHOH\text{-}(CH_2)_z\text{-}$ $CH_2\text{-}O\text{-}(C_nH_{2n}O)_x\text{-}H$ wobei in beiden Formeln y und z = 0 oder ganze Zahlen von 1 bis 18, p = 0, 1 oder 2 und die Summe (y+z+p) eine Zahl von 12 bis 18, x = 0 oder eine Zahl von 1 bis 30 und n eine ganze Zahl von 2 bis 4 sowie M = H oder Alkali-, insbesondere Natrium, Kalium, Lithium, Erdalkali- , insbesondere Magnesium, Calcium, Zink und/oder einem Ammoniumion, welches gegebenenfalls substituiert sein kann, insbesondere Mono-, Di-, Tri- oder Tetraammoniumionen mit C1 bis C4 Alkyl-, Alkenyl- oder Arylresten,
- sulfatierte Hydroxyalkylpolyethylen- und/oder Hydroxyalkylenpropylenglykolether der Formel $R^1\text{-}(CHOSO_3M)\text{-}CHR^3\text{-}(OCHR^4\text{-}CH_2)n\text{-}OR^2$ mit $R^1$, einem linearen Alkylrest mit 1 bis 24 C-Atomen, $R^2$ für einen linearen oder verzweigten, gesättigten Alkylrest mit 1 bis 24 C-Atomen, $R^3$ für Wasserstoff oder einen linearen Alkylrest mit 1 bis 24 C-Atomen, $R^4$ für Wasserstoff oder einen Methylrest und M für Wasserstoff, Ammonium, Alkylammonium, Alkanolammonium, worin die Alkyl- und Alkanolreste je 1 bis 4 C-Atome aufweisen, oder ein Metallatom ausgewählt aus Lithium, Natrium, Kalium, Calcium oder Magnesium und n für eine Zahl im Bereich von 0 bis 12 stehen und weiterhin die Gesamtzahl der in $R^1$ und $R^3$ enthaltenen C-Atome 2 bis 44 beträgt,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel,

$$R^1(OCH_2CH_2)_n\text{-}O\text{-}(PO\text{-}OX)\text{-}OR^2,$$

  in der $R^1$ bevorzugt für einen aliphatischen Kohlenwasserstoffrest mit 8 bis 30 Kohlenstoffatomen, $R^2$ für Wasserstoff, einen Rest $(CH_2CH_2O)_nR^2$ oder X, n für Zahlen von 1 bis 10 und X für Wasserstoff, ein Alkali- oder Erdalkalimetall oder $NR^3R^4R^5R^6$, mit $R^3$ bis $R^6$ unabhängig voneinander stehend für Wasserstoff oder einen $C_1$ bis $C_4$ - Kohlenwasserstoffrest, steht,
- sulfatierte Fettsäurealkylenglykolester der Formel

$$RCO(AlkO)_nSO_3M$$

in der RCO- für einen linearen oder verzweigten, aliphatischen, gesättigten und/oder ungesättigten Acylrest mit 6 bis 22 C-Atomen, Alk für $CH_2CH_2$, $CHCH_3CH_2$ und/oder $CH_2CHCH_3$, n für Zahlen von 0,5 bis 5 und M für ein Metall steht, wie Alkalimetall, insbesondere Natrium, Kalium, Lithium, Erdalkalimetall, insbesondere Magnesium, Calcium, Zink, oder Ammoniumion, wie $^+NR^3R^4R^5R^6$, mit $R^3$ bis $R^6$ unabhängig voneinander stehend für Wasserstoff oder einen C1 bis C4 - Kohlenwasserstoffrest,

- Monoglyceridsulfate und Monoglyceridethersulfate der Formel

$$R^8OC\text{-}(OCH_2CH_2)_x\text{-}OCH_2\text{-}[CHO(CH_2CH_2O)_yH]\text{-}CH_2O(CH_2CH_2O)_z\text{-}SO_3X,$$

in der $R^8CO$ für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate eingesetzt, in der $R^8CO$ für einen linearen Acylrest mit 8 bis 18 Kohlenstoffatomen steht,

- Amidethercarbonsäuren, $R^1\text{-CO-}NR^2\text{-}CH_2CH_2\text{-O-}(CH_2CH_2O)_nCH_2COOM$, mit $R^1$ als geradkettigen oder verzweigten Alkyl- oder Alkenylrest mit einer Zahl an Kohlenstoffatomen in der Kette von 2 bis 30, n steht für eine ganze Zahl von 1 bis 20 und $R^2$ steht für Wasserstoff, einen Methyl-, Ethyl-, Propyl-, Isopropyl-, n-Butyl-, t-Butyl- oder iso-Butylrest und M steht für Wasserstoff oder ein Metall wie Alkalimetall, insbesondere Natrium, Kalium, Lithium, Erdalkalimetall, insbesondere Magnesium, Calcium, Zink, oder ein Ammoniumion, wie $^+NR^3R^4R^5R^6$, mit $R^3$ bis $R^6$ unabhängig voneinander stehend für Wasserstoff oder einen C1 bis C4 - Kohlenwasserstoffrest. Derartige Produkte sind beispielsweise von der Firma Chem-Y unter der Produktbezeichnung Akypo® erhältlich.
- Acylglutamate der Formel XOOC-CH2CH2CH(C(NH)OR)-COOX, in der RCO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall, Ammonium, Alkylammonium, Alkanolammonium oder Glucammonium steht,
- Kondensationsprodukte aus einem wasserlöslichen Salz eines wasserlöslichen Eiweißhydrolysats mit einer C8 - C30 - Fettsäure. Solche Produkte sind unter dem Warenzeichen Lamepon®, Maypon®, Gluadin®, Hostapon® KCG oder Amisoft® seit langem im Handel erhältlich.
- Alkyl- und/oder Alkenyl-Oligoglykosidcarboxylate, -sulfate, -phosphate und/oder -isethionate,
- Acyllactylate und
- Hydroxymischethersulfate.

Sofern die milden anionischen Tenside Polyglycoletherketten enthalten, ist es ganz besonders bevorzugt, dass diese eine eingeengte Homologenverteilung aufweisen. Weiterhin ist es im Falle von milden anionischen Tensiden mit Polyglycolethereinheiten bevorzugt, dass die Zahl der Glykolethergruppen 1 bis 20 beträgt, bevorzugt 2 bis 15, besonders bevorzugt 2 bis 12. Besonders milde anionische Tenside mit Polyglykolethergruppen ohne eingeschränkte Homologenverteilung können beispielsweise auch erhalten werden, wenn einerseits die Zahl der Polyglykolethergruppen 4 bis 12 beträgt und als Gegenion Zn- oder Mg-ionen gewählt werden. Ein Beispiel hierfür ist das Handelsprodukt Texapon® ASV.

Nichtionische Tenside sind beispielsweise

- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,
- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,
- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,
- mit einem Methyl- oder $C_2$ - $C_6$ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- $C_{12}$-$C_{30}$-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol® - Typen (Cognis),

- alkoxilierte Triglyceride,
- alkoxilierte Fettsäurealkylester der Formel (Tnio-1)

$$R^1CO\text{-}(OCH_2CHR^2)_wOR^3 \quad \text{(Tnio-1)}$$

in der $R^1CO$ für einen linearen oder verzweigten, gesättigten und/oder ungesättigten Acylrest mit 6 bis 22 Kohlenstoffatomen, $R^2$ für Wasserstoff oder Methyl, $R^3$ für lineare oder verzweigte Alkylreste mit 1 bis 4 Kohlenstoffatomen und w für Zahlen von 1 bis 20 steht,
- Aminoxide,
- Hydroxymischether, $R^1O[CH_2CH(CH_3)O]_x(CH_2CHR^2O)_y[CH_2CH(OH)R^3]_z$ mit $R^1$ stehend für einen linearen oder verzweigten, gesättigten oder ungesättigten Alkyl- und/oder Alkenylrest mit 2 bis 30 C - Atomen, $R^2$ stehend für Wasserstoff, einen Methyl-, Ethyl-, Propyl- oder isoPropylrest, $R^3$ stehend für einen linearen oder verzweigten Alkylrest mit 2 bis 30 C - Atomen, x stehend für 0 oder eine Zahl von 1 bis 20, Y für eine Zahl von 1 bis 30 und z stehend für die Zahl 1, 2, 3 , 4 oder 5.
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside,
- Zuckertenside vom Typ der Fettsäure-N-alkylpolyhydroxyalkylamide,
- Fettsäureamidpolyglycolether, Fettaminpolyglycolether,
- Mischether bzw. Mischformale und Polysorbate.

Kationische Tenside der Formel (Tkat1-1) können zusätzlich verwendet werden.

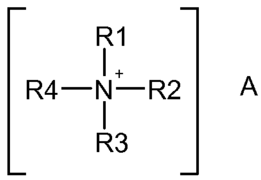

(Tkat1)

In der Formel (Tkat1) stehen R1, R2, R3 und R4 für jeweils unabhängig voneinander für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann. A steht für ein physiologisch verträgliches Anion, beispielsweise Halogenide wie Chlorid oder Bromid sowie Methosulfate.

Beispiele für Verbindungen der Formel (Tkat1) sind Lauryltrimehtylammoniumchlorid, Cetyltrimethylammoniumchlorid, Cetyltrimethylammoniumbromid, Cetyltrimethylammonium-methosulfat, Dicetyldimethylammoniumchlorid, Tricetylmethylammoniumchlorid, Stearyltrimethylammoniumchlorid, Distearyldimethylammoniumchlorid, Lauryldimethylbenzylammoniumchlorid, Behenyltrimethylammoniumchlorid, Behenyltrimethylammoniumbromid, Behenyltrimethylammoniummethosulfat.

Die Tenside werden in Mengen von 0,05 - 45 Gew.%, bevorzugt 0,1 - 30 Gew.% und ganz besonders bevorzugt von 0,5 - 25 Gew.%, bezogen auf das gesamte erfindungsgemäß verwendete Mittel, eingesetzt.

Erfindungsgemäß verwendbare Emulgatoren sind beispielsweise

- Anlagerungsprodukte von 4 bis 30 Mol Ethylenoxid und/oder 0 bis 5 Mol Pro pylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- $C_8$-$C_{22}$-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov®68,

- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine, sowohl aus tierischem Gewebe (Zoosterine, Cholesterin, Lanosterin) wie aus pflanzlichen Fetten (Phytosterine, Ergosterin, Stigmasterin, Sitosterin) oder aus Pilzen und Hefen (Mykosterine),
- Phospholipide (Lecithine, Phopshatidylcholine),
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls® PGPH).

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Mit besonderem Vorzug enthalten die erfindungsgemäßen Zusammensetzungen Fettstoffe (Fat) als weiteren Wirkstoff. Unter Fettstoffen (Fat) sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. Bevorzugt beträgt die Menge 0,5 - 10 Gew.%, wobei ganz besonders vorteilhaft Mengen von 1 - 5 Gew.% sein können.

Als Fettalkohole können eingesetzt werden gesättigte, ein- oder mehrfach ungesättigte, verzweigte oder unverzweigte Fettalkohole mit $C_6$ - $C_{30}$-, bevorzugt $C_{10}$ - $C_{22}$- und ganz besonders bevorzugt $C_{12}$ - $C_{22}$- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden. Die Fettalkohole werden in Mengen von 0,1 - 30 Gew.-%, bezogen auf die gesamte Zubereitung, bevorzugt in Mengen von 0,1 - 20 Gew.-% eingesetzt.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Die Gesamtmenge an Öl- und Fettkomponenten in den erfindungsgemäßen Mitteln beträgt üblicherweise 0,5 - 75 Gew.-%, bezogen auf das gesamte Mittel. Mengen von 0,5 - 35 Gew.-% sind erfindungsgemäß bevorzugt.

Ein weiterer erfindungsgemäßer synergistischer Wirkstoff in den erfindungsgemäßen Zusammensetzungen mit dem erfindungsgemäßen Wirkstoffkomplex sind Proteinhydrolysate und/oder dessen Derivate.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche

Gelatine Fabriken Stoess & Co), Lexein® (Inolex) und Kerasol® (Croda) vertrieben.

Weiterhin sind erfindungsgemäß bevorzugte pflanzliche Proteinhydrolysaten wie beispielsweise Soja-, Mandel-, Erbsen-, Moringa-, Kartoffel- und Weizenproteinhydrolysate. Solche Produkte sind beispielsweise unter den Warenzeichen Gluadin® (Cognis), DiaMin® (Diamalt), Lexein® (Inolex), Hydrosoy® (Croda), Hydrolupin® (Croda), Hydrosesame® (Croda), Hydrotritium® (Croda), Crotein® (Croda) und Puricare® LS 9658 von der Fa. Laboratoires Serobiologiques erhältlich.

Weitere erfindungsgemäß bevorzugte Proteinhydrolysate sind maritimen Ursprunges. Hierzu zählen beispielsweise Kollagenhydrolysate von Fischen oder Algen sowie Proteinhydrolysate von Muscheln bzw. Perlenhydrolysate. Beispiele für erfindungsgemäße Perlenextrakte sind die Handelsprodukte Pearl Protein Extract BG® oder Crodarom® Pearl.

Weiterhin sind kationisierte Proteinhydrolysate zu den Proteinhydrolysaten und deren Derivaten zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt.

Die Proteinhydrolysate sind in den Zusammensetzungen in Konzentrationen von 0,001 Gew.% bis zu 20 Gew.%, vorzugsweise von 0,05 Gew.% bis zu 15 Gew.% und ganz besonders bevorzugt in Mengen von 0,05 Gew.% bis zu 5 Gew.% enthalten.

Den Proteinhydrolysaten von der Wirkung her betrachtet nahe stehend und in einigen Fällen überlegen sind Aminosäuren und/oder Oligopetide. Erfindungsgemäß werden daher mit Vorzug Aminosäuren und/oder Oligopeptide als weitere Inhaltsstoffe verwendet. In der vorliegenden Anmeldung wird unter dem Begriff Aminosäure auch eine Struktur verstanden, die nur eine permanente kationische Gruppe im Molekül enthält wie beispielsweise Cholin. Weiterhin werden unter diesem Begriff Substanzen wie Carnitin oder Taurin verstanden, da sie wie die Aminosäuren in biologischen Systemen natürlicherweise vorkommen und sich in vielen Fällen wie Aminosäuren verhalten.

Erfindungsgemäße Aminosäuren sind ausgewählt aus Alanin, Arginin, Asparagin, Asparaginsäure, Cystein, Cystin, Citrullin, Glutaminsäure, Glutamin, Glycin, Histidin, Hydroxylysin, Hydroxyprolin, Isoleucin, Leucin, Lysin, Methionin, Phenylalanin, Prolin, Serin, Threonin, Thyroxin, Tryptophan, Tyrosin, Acetyltyrosin, Valin, Betain, Ornithin, 1,1-Dimethyl-Prolin, Hercynin ($N\alpha$,$N\alpha$,$N\alpha$-Trimethyl-L-histidinium-betain), Ergothionein (Thionein, 2-Mercapto-$N\alpha$, $N\alpha$, $N\alpha$-trimethyl-L-histidinium-betain), Carnitin, Taurin und Cholin sowie deren Mischungen. Es können erfindungsgemäß alle Arten von Isomeren, wie beispielsweise Diastereomere, Enantiomere, cis - trans-Isomere, optische Isomere, Konformationsisomere und Racemate verwendet werden.

Mit besonderem Vorzug werden Alanin, Arginin, Asparagin, Citrullin, Glutaminsäure, Glutamin, Glycin, Histidin, Hydroxylysin, Hydroxyprolin, Isoleucin, Leucin, Lysin, Prolin, Serin, Betain, Ornithin, Acetyltyrosin, 1,1-Dimethyl-Prolin, Carnitin, Taurin, Cholin sowie deren Mischungen verwendet.

Ganz besonders bevorzugt werden Arginin, Citrullin, Glutamin, Glycin, Histidin, Lysin, Prolin, Serin, Betain, Ornithin, Carnitin, Taurin, Acetyltyrosin sowie deren Mischungen verwendet.

Höchst bevorzugt werden Arginin, Citrullin, Glutamin, Histidin, Lysin, Acetyltyrosin, Ornithin, Carnitin und Taurin sowie deren Mischungen verwendet.

Allerhöchst bevorzugt sind Arginin, Citrullin, Glutamin, Acetyltyrosin, Ornithin, Carnitin und Taurin sowie die Mischungen aus Arginin und Taurin, Glutamin und Taurin, Glutamin und Carnitin, Arginin und Glutamin, Carnitin und Taurin, sowie die Mischungen aus Arginin, Carnitin und Taurin, Glutamin, Carnitin und Taurin und Arginin, Acetyltyrosin, Ornithin und Citrullin.

Oligopeptide im Sinne der vorliegenden Anmeldung sind durch Peptid-Bindungen Säureamid-artig verknüpfte Kondensationsprodukte von Aminosäuren, die mindestens 3 und maximal 25 Aminosäuren umfassen. In erfindungsgemäß bevorzugten Haarbehandlungsmitteln umfaßt das Oligopeptid 5 bis 15 Aminosäuren, vorzugsweise 6 bis 13 Aminosäuren, besonders bevorzugt 7 bis 12 Aminosäuren und insbesondere 8, 9 oder 10 Aminosäuren.

Ein höchst bevorzugtes Oligopeptid weist die Sequenz Glu-Glu-Glu auf. Je nachdem, ob weitere Aminosäuren an die Sequenz Glu-Glu-Glu gebunden sind und je nach Art dieser Aminosäuren kann die Molmasse des in den erfindungsgemäßen Mitteln enthaltenen Oligopeptids variieren. Erfindungsgemäß bevorzugte Haarbehandlungsmittel sind dadurch gekennzeichnet, daß das Oligopeptid eine Molmasse von 650 bis 3000 Da, vorzugsweise von 750 bis 2500 Da, besonders bevorzugt von 850 bis 2000 Da und insbesondere von 1000 bis 1600 Da aufweist.

Wie aus der bevorzugten Anzahl von Aminosäuren in den Oligopeptiden und dem bevorzugten Molmassenbereich zu ersehen ist, werden vorzugsweise Oligopeptide eingesetzt, die nicht allein aus den drei Glutaminsäuren bestehen, sondern weitere, an diese Sequenz gebundene Aminosäuren aufweisen. Diese weiteren Aminosäuren sind vorzugsweise aus bestimmten Aminosäuren ausgewählt, während bestimmte andere Vertreter erfindungsgemäß weniger bevorzugt sind. So ist es bevorzugt, wenn die in den erfindungsgemäßen Mitteln eingesetzten Oligopeptide kein Methionin enthalten.Weiter bevorzugt ist es, wenn die in den erfindungsgemäßen Mitteln eingesetzten Oligopeptide kein Cystein und/oder Cystin enthalten. Weiter bevorzugt ist es, wenn die in den erfindungsgemäßen Mitteln eingesetzten Oligopeptide keine Asparaginsäure und/oder Asparagin enthalten. Weiter bevorzugt ist es, wenn die in den erfindungsgemäßen Mitteln eingesetzten Oligopeptide kein Serin und/oder Threonin enthalten.

Demgegenüber ist es bevorzugt, wenn die in den erfindungsgemäßen Mitteln eingesetzten Oligopeptide Tyrosin enthalten. Weiter bevorzugt ist es, wenn die in den erfindungsgemäßen Mitteln eingesetzten Oligopeptide Leucin enthalten. Weiter bevorzugt ist es, wenn die in den erfindungsgemäßen Mitteln eingesetzten Oligopeptide Isoleucin enthalten. Weiter bevorzugt ist es, wenn die in den erfindungsgemäßen Mitteln eingesetzten Oligopeptide Arginin enthalten. Weiter bevorzugt ist es, wenn die in den erfindungsgemäßen Mitteln eingesetzten Oligopeptide Valin enthalten. Besonders bevorzugte Oligopeptide bzw. in den bevorzugten Oligopeptiden enthaltene Aminosäuresequenzen werden nachstehend beschrieben:

Ein besonders bevorzugtes Oligopeptid enthält zusätzlich Tyrosin, das vorzugsweise über seine Säurefunktion an die Glu-Glu-Glu-Sequenz gebunden ist. Erfindungsgemäß bevorzugte Haarbehandlungsmittel sind daher dadurch gekennzeichnet, daß das in ihnen enthaltene Oligopeptid mindestens eine Aminosäuresequenz Tyr-Glu-Glu-Glu aufweist, wobei die Amino-Gruppe frei oder protoniert und die Carboxy-Gruppen frei oder deprotoniert vorliegen können.

Ein weiteres besonders bevorzugtes Oligopeptid enthält zusätzlich Isoleucin, das vorzugsweise über seine Aminofunktion an die Glu-Glu-Glu-Sequenz gebunden ist. Erfindungsgemäß bevorzugte Haarbehandlungsmittel sind daher dadurch gekennzeichnet, daß das in ihnen enthaltene das Oligopeptid mindestens eine Aminosäuresequenz Glu-Glu-Glu-Ile aufweist, wobei die Amino-Gruppe frei oder protoniert und die Carboxy-Gruppen frei oder deprotoniert vorliegen können. Oligopeptide, die beide vorgenannten Aminosäuren (Tyrosin und Isoleucin) aufweisen, sind erfindungsgemäß bevorzugt. Besonders bevorzugt sind dabei erfindungsgemäße Haarbehandlungsmittel, bei denen das in ihnen enthaltene Oligopeptid mindestens eine Aminosäuresequenz Tyr-Glu-Glu-Glu-Ile aufweist, wobei die Amino-Gruppe frei oder protoniert und die Carboxy-Gruppen frei oder deprotoniert vorliegen können.

Weiter bevorzugte Oligopeptide enthalten zusätzlich Arginin, das vorzugsweise an Isoleucin gebunden vorliegt. Ein höchst bevorzugtes Oligopeptid ist unter der Handelsbezeichnung ProSina® von der Fa. Croda im Handel erhältlich.

Die erfindungsgemäßen Haarbehandlungsmittel enthalten die ausgewählten Aminosäuren und/oder die ausgewählten Oligopeptide wie zuvor beschrieben in einer Gesamtmenge - bezogen auf das gesamte Mittel - in einer Menge von 0,0001 bis 10,0 Gew.-%, besonders bevorzugt von 0,0001 bis 7,0 Gew.-%, ganz besonders bevorzugt von 0,0001 bis 5,0 Gew.-%.

Eine weitere bevorzugte Gruppe von Inhaltsstoffen der erfindungsgemäßen Zusammensetzungen mit dem erfindungsgemäßen Wirkstoffkomplex sind Vitamine, Provitamine oder Vitaminvorstufen. Vitamine, Pro-Vitamine und Vitaminvorstufen sind dabei besonders bevorzugt, die den Gruppen A, B, C, E, F und H zugeordnet werden.

Zur Gruppe der als Vitamin A bezeichneten Substanzen gehören das Retinol (Vitamin $A_1$) sowie das 3,4-Didehydroretinol (Vitamin $A_2$). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.:

Vitamin $B_1$ (Thiamin)

Vitamin $B_2$ (Riboflavin)

Vitamin $B_3$. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.

Vitamin $B_5$ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Pantothensäure wird bevorzugt als Derivat in Form der stabileren Calciumsalze und Natriumsalze (Ca-Pantothenat, Na-Pantothenat) in der vorliegenden Erfindung eingesetzt.

Vitamin $B_6$ (Pyridoxin sowie Pyridoxamin und Pyridoxal).

Die genannten Verbindungen des Vitamin B -Typs insbesondere Vitamin $B_3$, $B_5$ und $B_6$, sind in den erfindungsgemäßen Mitteln bevorzugt in Mengen von 0,05 - 10 Gew.-%, bezogen auf das gesamte Mittel, enthalten. Mengen von

0,1-5 Gew.-% sind besonders bevorzugt.

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere $\alpha$-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Zusammensetzungen Vitamine, Provitamine und Vitaminvorstufen aus den Gruppen A, B, E und H. Panthenol, Pantolacton, Pyridoxin und seine Derivate sowie Nicotinsäureamid und Biotin sind besonders bevorzugt.

Eine besonders bevorzugte Gruppe von Inhaltsstoffen in den erfindungsgemäßen kosmetischen Zusammensetzungen sind die im folgenden genannten Betaine: Carnitin, Carnitintartrat, Carnitin Magnesiumcitrat, Acetylcarnitin, Betalaine, 1,1-Dimethyl-Prolin, Cholin, Cholinchlorid, Cholinbitartrat, Cholindihydrogencitrat und die in der Literatur als Betain bezeichnete Verbindung N,N,N-trimethylglycin.

Bevorzugt werden Carnitin, Histidin, Cholin sowie Betain verwendet. In einer besonders bevorzugten Ausführungsform der Erfindung wird als Wirkstoff L-Carnitintartrat eingesetzt.

In einer weiteren erfindungsgemäß bevorzugten Ausführungsform enthalten die erfindungsgemäßen Zusammensetzungen Biochinone. In den erfindungsgemäßen Mitteln sind unter geeigneten Biochinonen ein oder mehrere Ubichinon(e) und/oder Plastochinon(e) zu verstehen. Die erfindungsgemäß bevorzugten Ubichinone weisen die folgende Formel auf:

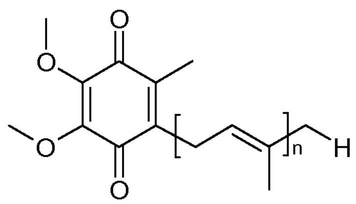

mit n = 6, 7, 8, 9 oder 10.

Das Coenzym Q-10 ist hierbei am bevorzugtesten.

Bevorzugte erfindungsgemäße Zusammensetzungen enthalten Purin und/oder Purinderivate in engeren Mengenbereichen. Hier sind erfindungsgemäß bevorzugte kosmetische Mittel dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten. Erfindungsgemäß bevorzugte kosmetische Mittel sind dadurch gekennzeichnet, daß sie Purin, Adenin, Guanin, Harnsäure, Hypoxanthin, 6-Purinthiol, 6-Thioguanin, Xanthin, Coffein, Theobromin oder Theophyllin enthalten. In haarkosmetischen Zubereitungen ist Coffein am bevorzugtesten.

In einer weiteren bevorzugten Ausführungsform der vorliegenden Erfindung enthält das kosmetische Mittel Ectoin ((*S*)-2-Methyl-1,4,5,6-tetrahydro-4-pyrimidincarbonsäure.

Erfindungsgemäß besonders bevorzugt sind Mittel, die - bezogen auf ihr Gewicht - 0,00001 bis 10,0 Gew.-%, vorzugsweise 0,0001 bis 5,0 Gew.-% und insbesondere 0,001 bis 3 Gew.-% der Wirkstoffe aus der Gruppe, die gebildet wird von Carnitin, Coenzym Q-10, Ectoin, ein Vitamin der B - Reihe, einem Purin und deren Derivaten oder physiologisch vertretbaren Salze enthalten.

Ein ganz besonders bevorzugter Pflegezusatz in den erfindungsgemäßen Haarbehandlungsmitteln ist Taurin. Unter Taurin wird ausschließlich 2-Aminoethansulfonsäure und unter einem Derivat die explizit genannten Derivate des Taurines verstanden. Unter den Derivaten des Taurines werden N-Monomethyltaurin, N,N-Dimethyltaurin, Taurinlysylat, Taurintartrat, Taurinornithat, Lysyltaurin und Ornithyltaurin verstanden.

Besonders bevorzugt sind erfindungsgemäße Mittel, die - bezogen auf ihr Gewicht - 0,0001 bis 10,0 Gew.-%, vorzugsweise 0,0005 bis 5,0 Gew.-%, besonders bevorzugt 0,001 bis 2,0 Gew.-% und insbesondere 0,001 bis 1,0 Gew.-% Taurin und/oder eines Derivates des Taurines enthalten. Die Wirkung der erfindungsgemäßen Zusammensetzungen kann weiterhin durch eine 2-Pyrrolidinon-5-carbonsäure und deren Derivate (J) gesteigert werden. Bevorzugt sind die

Natrium-, Kalium-, Calcium-, Magnesium- oder Ammoniumsalze, bei denen das Ammoniumion neben Wasserstoff eine bis drei $C_1$- bis $C_4$-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf das gesamte Mittel, besonders bevorzugt 0,1 bis 5, und insbesondere 0,1 bis 3 Gew.%.

Durch die Verwendung von Pflanzenextrakten als Pflegestoffe können die erfindungsgemäßen Haarbehandlungsmittel besonders naturnah und dennoch sehr effektiv in ihrer Pflegeleistung formuliert werden. Gegebenenfalls kann dabei sogar auf ansonsten übliche Konservierungsmittel verzichtet werden. Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Baldrian, Wiesenschaumkraut, Quendel, Schafgarbe, Thymian, Melisse, Hauhechel, Huflattich, Eibisch, Meristem, Ginseng, Kaffee, Kakao, Moringa, Ingwerwurzel und ayurvedische Pflanzenextrakte wie beispielsweise Aegle Marmelos (Bilwa), Cyperus Rotundus (Nagar Motha), Emblica Officinalis (Amalki), Morida Citrifolia (Ashyuka), Tinospora Cordifolia (Guduchi), Santalum album, (Chandana), Crocus Sativus (Kumkuma), Cinnamonum Zeylanicum und Nelumbo Nucifera (Kamala), Süßgräser wie Weizen, Gerste, Roggen, Hafer, Dinkel, Mais, die verschiedenen Sorten der Hirse (Rispenhirse, Fingerhirse, Kolbenhirse als Beispiele), Zuckerrohr, Weidelgras, Wiesenfuchsschwanz, Glatthafer, Straußgras, Wiesenschwingel, Pfeifengras, Bambus, Baumwollgras, Lampenputzergräser, Andropogonodeae (Imperata Cylindrica auch Flammengras oder Cogon Gras genannt), Büffelgras, Schlickgräser, Hundszahngräser, Liebesgräser, Cymbopogon (Zitronengras), Oryzeae (Reis), Zizania (Wildreis), Strandhafer, Staudenhafer, Honiggräser, Zittergräser, Rispengräser, Quecken und Echinacea, insbesondere Echinacea purpurea (L.) Moench, aller Arten von Wein sowie Perikarp von Litchie chinensis bevorzugt.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Gelegentlich kann es erforderlich sein anionische Polymere zu verwenden. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfon-säure, das beispielsweise unter der Bezeichnung Rheothik®11-80 im Handel erhältlich ist.

Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein solches Polymer ist in dem Handelsprodukt Sepigel®305 der Firma SEPPIC enthalten.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze® QM im Handel erhältlich.

Die anionischen Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Die erfindungsgemäßen Mittel können in einer weiteren Ausführungsform nichtionogene Polymere enthalten.

Geeignete nichtionogene Polymere sind beispielsweise:

- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) und Natrosol®-Typen (Hercules) vertrieben werden.
- Stärke und deren Derivate, insbesondere Stärkeether, beispielsweise Structure® XL (National Starch), eine multifunktionelle, salztolerante Stärke;
- Schellack

- Polyvinylpyrrolidone, wie sie beispielsweise unter der Bezeichnung Luviskol® (BASF) vertrieben werden.

Die nichtionischen Polymere sind in den erfindungsgemäßen Zusammensetzungen bevorzugt in Mengen von 0,05 bis 10 Gew.-%, bezogen auf das gesamte Mittel, enthalten. Mengen von 0,1 bis 5 Gew.-% sind besonders bevorzugt.
In einer weiteren Ausführungsform sollten die erfindungsgemäßen Mittel zusätzlich mindestens einen UV-Lichtschutzfilter enthalten. UVB-Filter können öllöslich oder wasserlöslich sein.
Als öllösliche Substanzen sind z.B. zu nennen:

- 3-Benzylidencampher, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylben-zylester, Salicylsäurehomomenthylester;
- Derivate des Benzophenons, vorzugsweise 2-Hydroxy-4-methoxybenzophenon, 2-Hydroxy-4-meth-oxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion;

Als wasserlösliche Substanzen kommen in Frage:

- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sul-fonsäure und ihre Salze;
- Sulfonsäurederivate des 3-Benzylidencamphers, wie z.B. 4-(2-Oxo-3-bornylidenmethyl)benzolsul-fonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Pigmente, insbesondere feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen.
Ferner können die kosmetischen Mittel weitere Wirk-, Hilfs- und Zusatzstoffe enthalten, wie beispielsweise

- Strukturanten wie Maleinsäure und Milchsäure,
- Quellmittel wie Harnstoff, Allantoin, Carbonate oder Hydantoin,
- Dimethylisosorbid und Cyclodextrine,
- Farbstoffe zum Anfärben des Mittels,
- Komplexbildner wie EDTA, NTA, β-Alanindiessigsäure und Phosphonsäuren,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, $N_2O$, Dimethylether, $CO_2$ und Luft,
- Antioxidantien,
- Parfümöle, Duftstoffe und Riechstoffe.

Bezüglich weiterer fakultativer Komponenten sowie die eingesetzten Mengen dieser Komponenten wird ausdrücklich auf die dem Fachmann bekannten einschlägigen Handbücher verwiesen.
Ein weiterer Erfindungsgegenstand ist daher ein Verfahren zur Haarbehandlung, in dem ein Haarbehandlungsmittel gemäß Anspruch 1 auf das Haar aufgetragen wird und nach einer Einwirkungszeit vom Haar gespült wird.

Die Einwirkungszeit beträgt bevorzugt wenige Sekunden bis 100 Minuten, besonders bevorzugt 1 bis 50 Minuten und ganz besonders bevorzugt 1 bis 30 Minuten.

Erfindungsgemäß ist weiterhin ein Verfahren, bei welchem ein kosmetisches Mittel gemäß Anspruch 1 auf das Haar aufgetragen wird und dort verbleibt. Unter "auf dem Haar verbleiben" wird erfindungsgemäß verstanden, dass das Mittel nicht unmittelbar nach dessen Anwendung wieder aus dem Haar ausgespült wird. Vielmehr verbleibt das Mittel in diesem Falle mehr als 100 Minuten bis hin zur nächsten Haarwäsche auf dem Haar.

Schließlich ist erfindungsgemäß die Verwendung einer Zusammensetzung wie zuvor beschrieben zur Verringerung und/oder Verzögerung des Schuppens der Kopfhaut.

Die folgenden Beispiele sollen den Gegenstand der vorliegenden Erfindung erläutern ohne ihn jedoch zu beschränken.

Beispiele

Alle Mengenangaben sind, soweit nicht anders vermerkt, Gewichtsteile. Die folgenden Rezepturen wurden unter Anwendung bekannter Herstellungsverfahren bereitgestellt

Haarkuren, auch in Schaumform anwendbar:

| | K1 | K2 | K3 | K4 | K5 | K6 | K7 | K8 | K9 | K10 | K11 |
|---|---|---|---|---|---|---|---|---|---|---|---|
| Polymer JR 400 | 0,2 | 0,2 | 0,2 | 0,2 | 0,2 | 0,2 | 0,2 | 0,2 | 0,2 | 0,2 | 0,2 |
| Armocare VGH 70 | 0,5 | 0,5 | 0,5 | 0,5 | 0,5 | 0,5 | 0,5 | 0,5 | 0,5 | 0,5 | 0,5 |
| Stearamidopropyl Dimethylamine | 0,5 | 0,5 | 0,5 | 0,5 | 0,5 | 0,5 | 0,5 | 0,5 | 0,5 | 0,5 | 0,5 |
| PVP/VA Copolymer 60/40 | 1,0 | 1,0 | 1,0 | 1,0 | 1,0 | 1,0 | 1,0 | 1,0 | 1,0 | 1,0 | 1,0 |
| Selenhaltiges Pflanzenöl | 1,0 | --- | --- | --- | --- | --- | --- | --- | --- | --- | --- |
| Selenhaltigen Pflanzenextrakt | --- | 2,0 | --- | --- | --- | --- | --- | --- | --- | --- | --- |
| Ketoconazol | --- | --- | --- | --- | --- | 0,5 | --- | --- | --- | --- | 0,5 |
| Zink-Pyrithion | --- | --- | --- | --- | --- | --- | 0,5 | --- | --- | 0,5 | --- |
| Octopirox | --- | --- | --- | --- | --- | --- | --- | 0,5 | --- | 0,5 | 0,5 |
| Climbazol | --- | --- | --- | --- | --- | --- | --- | --- | 0,5 | 0,5 | 0,5 |
| SeS | --- | --- | 0,5 | --- | --- | --- | --- | --- | --- | --- | --- |
| Se, kolloidal | --- | --- | --- | 0,5 | --- | --- | --- | --- | --- | --- | --- |
| S, kolloidal | --- | --- | --- | --- | 0,5 | --- | --- | --- | --- | --- | --- |
| Panthenol | 0,2 | 0,2 | 0,2 | 0,2 | 0,2 | 0,2 | 0,2 | 0,2 | 0,2 | 0,2 | 0,2 |
| Cetrimonium Chloride | 5,0 | 5,0 | 5,0 | 5,0 | 5,0 | 5,0 | 5,0 | 5,0 | 5,0 | 5,0 | 5,0 |
| Ceteareth-25 | 0,3 | 0,3 | 0,3 | 0,3 | 0,3 | 0,3 | 0,3 | 0,3 | 0,3 | 0,3 | 0,3 |
| Proteinhydrolysat | 0,2 | 0,2 | 0,2 | 0,2 | 0,2 | 0,2 | 0,2 | 0,2 | 0,2 | 0,2 | 0,2 |
| Dow Corning 193 | 0,2 | 0,2 | 0,2 | 0,2 | 0,2 | 0,2 | 0,2 | 0,2 | 0,2 | 0,2 | 0,2 |
| PEG-8 PG-Coco-Glucoside Dimethicone | 0,5 | 0,5 | 0,5 | 0,5 | 0,5 | 0,5 | 0,5 | 0,5 | 0,5 | 0,5 | 0,5 |
| Coco Betaine | 0,5 | 0,5 | 0,5 | 0,5 | 0,5 | 0,5 | 0,5 | 0,5 | 0,5 | 0,5 | 0,5 |
| Wasser, Konservierung und ggf. Parfümöle | ad 100 | ad 100 | ad 100 | ad 100 | ad 100 | ad 100 | ad 100 | ad 100 | ad 100 | ad 100 | ad 100 |

Die pH - Werte aller Rezepturen wurde eingestellt auf 2 bis 6.

Zur Applikation als Schaum wird die betreffende Rezeptur entweder mit einem Treibgas in einem Aerosolbehälter abgefüllt oder aus einer Pumpflasche mit einem entsprechenden Pumpenaufsatz, wie beispielsweise Airfoamer, als Schaum ausgebracht.

Zur Anwendung als Haarkur oder Creme werden zu den o.a Rezepturen Fettalkohol wie Cetylstearylalkohol und/oder Ethylenglykoldistearat und /oder Glycerinmonostearat in Mengen von 0,2 bis 5,0 Gew.% zugegeben.

Shampoo: Die pH - Werte aller Rezepturen wurden eingestellt auf 4,5 bis 5,8.

| | S1 | S2 | S3 | S4 | S5 | S6 | S7 | S8 | S9 | S10 | S11 |
|---|---|---|---|---|---|---|---|---|---|---|---|
| Texapon® N70 | 15,0 | 15,0 | 15,0 | 15,0 | 15,0 | 15,0 | 15,0 | 15,0 | 15,0 | 15,0 | 15,0 |
| Arlypon® F | 0,15 | 0,15 | 0,15 | 0,15 | 0,15 | 0,15 | 0,15 | 0,15 | 0,15 | 0,15 | 0,15 |
| Antil® 141 | 0,15 | 0,15 | 0,15 | 0,15 | 0,15 | 0,15 | 0,15 | 0,15 | 0,15 | 0,15 | 0,15 |
| Disodium Cocoamphodiacetate | 6,0 | 6,0 | 6,0 | 6,0 | 6,0 | 6,0 | 6,0 | 6,0 | 6,0 | 6,0 | 6,0 |
| Polyquaternium-10 | 0,5 | 0,5 | 0,5 | 0,5 | 0,5 | 0,5 | 0,5 | 0,5 | 0,5 | 0,5 | 0,5 |
| Selenhaltiges Pflanzenöl | 1,0 | --- | --- | --- | --- | --- | --- | --- | --- | --- | --- |
| Selenhaltiger Pflanzenextrakt | --- | 2,0 | --- | --- | --- | --- | --- | --- | --- | --- | --- |
| Ketoconazol | --- | --- | --- | --- | --- | 0,5 | --- | --- | --- | --- | 0,5 |
| Zink-Pyrithion | --- | --- | --- | --- | --- | --- | 0,5 | --- | --- | 0,5 | --- |
| Octopirox | --- | --- | --- | --- | --- | --- | --- | 0,5 | --- | 0,5 | 0,5 |
| Climbazol | --- | --- | --- | --- | --- | --- | --- | --- | 0,5 | 0,5 | 0,5 |
| SeS | --- | --- | 0,5 | --- | --- | --- | --- | --- | --- | --- | --- |
| Se, kolloidal | --- | --- | --- | 0,5 | --- | --- | --- | --- | --- | --- | --- |
| S, kolloidal | --- | --- | --- | --- | 0,5 | --- | --- | --- | --- | --- | --- |
| Cetiol ® HE | 3,0 | 3,0 | 3,0 | 3,0 | 3,0 | 3,0 | 3,0 | 3,0 | 3,0 | 3,0 | 3,0 |
| Panthenol | 0,2 | 0,2 | 0,2 | 0,2 | 0,2 | 0,2 | 0,2 | 0,2 | 0,2 | 0,2 | 0,2 |
| Dow Corning ® 193 | 0,1 | 0,1 | 0,1 | 0,1 | 0,1 | 0,1 | 0,1 | 0,1 | 0,1 | 0,1 | 0,1 |
| PEG-8 PG-Coco-Glucoside Dimethicone | 1,0 | 1,0 | 1,0 | 1,0 | 1,0 | 1,0 | 1,0 | 1,0 | 1,0 | 1,0 | 1,0 |
| Proteinhydrolysat | 0,5 | 0,5 | 0,5 | 0,5 | 0,5 | 0,5 | 0,5 | 0,5 | 0,5 | 0,5 | 0,5 |
| Cremophor® HRE 60 | 0.5 | 0.5 | 0.5 | 0.5 | 0.5 | 0.5 | 0.5 | 0.5 | 0.5 | 0.5 | 0.5 |
| Wasser, Konservierung und ggf. Parfümöle | ad 100 | ad 100 | ad 100 | ad 100 | ad 100 | ad 100 | ad 100 | ad 100 | ad 100 | ad 100 | ad 100 |

## Patentansprüche

1. Kosmetische Zusammensetzung enthaltend in einem kosmetischen Träger - jeweils bezogen auf das Gewicht der gesamten Zusammensetzung -

   a) mindestens einen Antischuppenwirkstoff in einer Gesamtmenge von 0,01 bis 10,0 Gew.%, ausgewählt aus

      i. Zink-Pyrithion,
      ii. Climbazol,

iii. Octopirox,
iv. Ketoconazol,
v. Selendisulfid,
vi. selenhaltige Pflanzenöle und
vii. selenhaltige Pflanzenextrakte sowie deren Mischungen und

b) mindestens ein Silikon enthaltend Zuckerstrukturen der folgenden Formel,

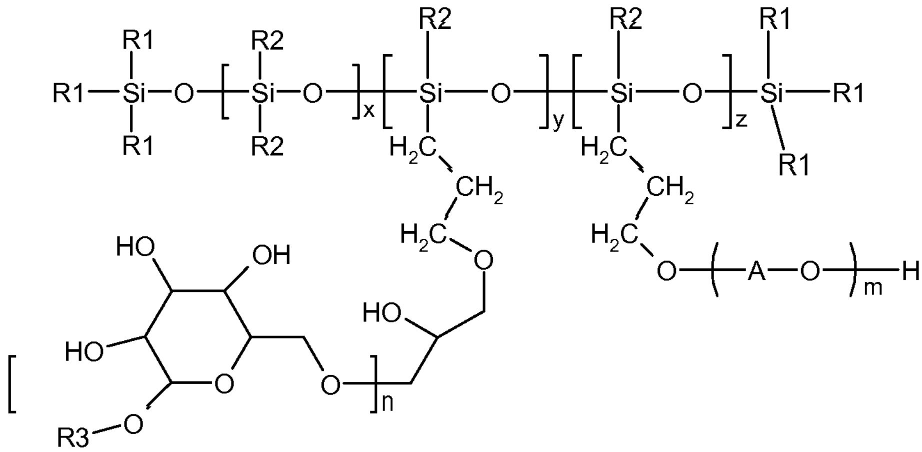

in welcher A steht für eine Gruppe ausgewählt aus -CH2CH2-, -CH2CH2CH2- oder - CH2CH2CH2CH2- oder Mischungen dieser Gruppen, die Reste R1, R2 und R3 unabhängig voneinander stehen für eine Methyl, Ethyl, Propyl, iso-Propyl, Hydroxy, Methoxy oder Ethoxygruppe, x, y und z stehen für jeweils für eine ganze Zahl von 1 bis 1000, n und m stehen unabhängig voneinander jeweils für eine ganze Zahl von 1 bis 100, in einer Gesamtmenge von 0,01 bis 5,0 Gew.%.

2. Kosmetische Zusammensetzung laut Anspruch 1 dargestellt, **dadurch gekennzeichnet, dass** weiterhin mindestens eine quartäre Ammoniumverbindung in einer Gesamtmenge von 0,1 bis 10,0 Gew.% bezogen auf das Gewicht der gesamten Zusammensetzung, ausgewählt aus mindestens einer der Gruppen der

i) der Esterquats und/oder
ii) der quarternären Imidazoline der Formel (Tkat2),

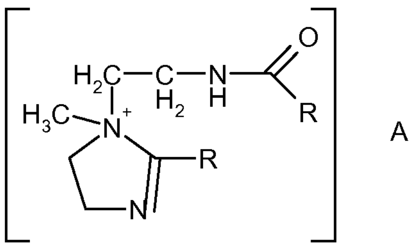

in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
iii) der Amine und/oder kationisierten Amine und/oder
iv) Poly(methacryloyloxyethyltrimethylammoniumverbindungen) und/oder;
v) quaternisierten Cellulose-Derivaten, insbesondere Polyquaternium 10, Polyquaternium-24, Polyquaternium-27, Polyquaternium-67, Polyquaternium-72, und/oder
vi) kationischen Alkylpolyglycosiden und/oder
vii) kationisiertem Honig und/oder
viii) kationischen Guar-Derivaten und/oder
ix) Chitosan und/oder
x) polymeren Dimethyldiallylammoniumsalzen und deren Copolymeren mit Estern und Amiden von Acrylsäure

und Methacrylsäure, insbesondere Polyquaternium-7 und/oder
xi) Copolymeren des Vinylpyrrolidons mit quaternierten Derivaten des Dialkylaminoalkylacrylats und -methacrylats, insbesondere Polyquaternium-11 und/oder
xii) Vinylpyrrolidon-Vinylimidazoliummethochlorid-Copolymeren, insbesondere Polyquaternium-16 und/oder
xiii) quaterniertem Polyvinylalkohol und/oder
xiv) Polyquaternium-74,

sowie Mischungen hiervon.

3. Kosmetische Zusammensetzung gemäss den vorstehenden Ansprüchen, **dadurch gekennzeichnet, dass** das Antischuppenmittel ausgewählt ist aus Zink-Pyrithion und/oder Climbazol und/oder Octopirox und deren Mischungen.

4. Kosmetische Zusammensetzung gemäss den vorstehenden Ansprüchen, **dadurch gekennzeichnet, dass** die quatäre Ammoniumverbindung ausgewählt ist aus Stearamidopropyldimethylamine und/oder Distearoylethyl Hydroxyethylmonium Methosulfate und/oder Dicocoyl Hydroxyethylmonium Methosulfate und/oder Dipalmitoylethyl Dimoniumchloride und/oder Quaternium-27 und/oder Quaternium-91 und/oder Behenoyl PG -Trimoniumchloride.

5. Kosmetische Zusammensetzung gemäss den vorstehenden Ansprüchen, **dadurch gekennzeichnet, dass** das Silikon enthaltend Zuckerstrukturen PEG-8 PG-Coco-Glucoside Dimethicone ist.

6. Kosmetische Zusammensetzung gemäss den vorstehenden Ansprüchen, **dadurch gekennzeichnet, dass** als weiteres kationisches Tensid Behentrimoniumchloride und/oder Cetyltrimethylammoniumchlorid verwendet wird.

7. Kosmetische Zusammensetzung gemäss den vorstehenden Ansprüchen, **dadurch gekennzeichnet, dass** weiterhin mindestens ein zwitterionische und/oder amphoteres Tensid enthalten ist.

8. Kosmetische Zusammensetzung gemäss den vorstehenden Ansprüchen, dass das zwitterionische und/oder amphotere Tensid ausgewählt ist aus Cocamidopropylbetain und/oder Coco Betaine.

9. Kosmetische Zusammensetzung gemäss den vorstehenden Ansprüchen, **dadurch gekennzeichnet, dass** zusätzlich mindestens eine Aminosäure ausgewählt aus Alanin, Arginin, Asparagin, Asparaginsäure, Cystein, Cystin, Citrullin, Glutaminsäure, Glutamin, Glycin, Histidin, Hydroxylysin, Hydroxyprolin, Isoleucin, Leucin, Lysin, Methionin, Phenylalanin, Prolin, Serin, Threonin, Thyroxin, Tryptophan, Tyrosin, Acetyltyrosin, Valin, Betain, Ornithin, 1,1-Dimethyl-Prolin, Hercynin und Ergothionein sowie deren Mischungen enthalten ist.

10. Kosmetische Zusammensetzung gemäss den vorstehenden Ansprüchen, **dadurch gekennzeichnet, dass** zusätzlich mindestens ein Oligopeptid umfassend mindestens 3 bis höchstens 25 Aminosäuren. Kosmetische Zusammensetzung wie zuvor dargestellt, **dadurch gekennzeichnet, dass** zusätzlich mindestens ein Oligopeptid und enthaltend die Sequenz Glu-Glu-Glu.

11. Kosmetische Zusammensetzung gemäss den vorstehenden Ansprüchen, **dadurch gekennzeichnet, dass** zusätzlich mindestens ein Wirkstoff ausgewählt aus der Gruppe, die gebildet wird von Carnitin, Taurin, Coenzym Q-10, Ectoin, einem Purin und dessen Derivaten, insbesondere Coffein, oder deren physiologisch vertretbaren Salze, sowie einem Vitamin der B - Reihe, insbesondere Panthenol, enthalten ist.

12. Kosmetische Zusammensetzung gemäss den vorstehenden Ansprüchen, **dadurch gekennzeichnet, dass** zusätzlich mindestens ein aminofunktionales Silikon zusätzlich enthalten ist.

13. Kosmetische Zusammensetzung gemäss den vorstehenden Ansprüchen, **dadurch gekennzeichnet, dass** das aminofunktionale Silikon ausgewählt ist aus den 4-morpholinomethyl-substituierten Silikonen.

14. Verfahren zur Behandlung keratinischer Fasern **dadurch gekennzeichnet, dass** eine kosmetische Zusammensetzung gemäss den vorstehenden Ansprüchen auf die keratinischen Fasern aufgetragen wird und nach einer Einwirkzeit von wenigen Sekunden bis hin zu 45 Minuten wieder ausgespült wird.

15. Verwendung einer kosmetischen Zusammensetzung gemäss den vorstehenden Ansprüchen 1-13 zur Verminderung der Bildung von Schuppen auf der Kopfhaut.

## Claims

1. A cosmetic composition containing, in a cosmetic carrier and in each case based on the weight of the overall composition,

   a) at least one anti-dandruff active ingredient in a total amount of from 0.01 to 10.0 wt.%, selected from:

      i. zinc pyrithione,
      ii. climbazole,
      iii. Octopirox,
      iv. ketoconazole,
      v. selenium disulfide,
      vi. selenium-containing plant oils and
      vii. selenium-containing plant extracts and mixtures thereof, and

   b) at least one sugar-structure-containing silicone of the following formula:

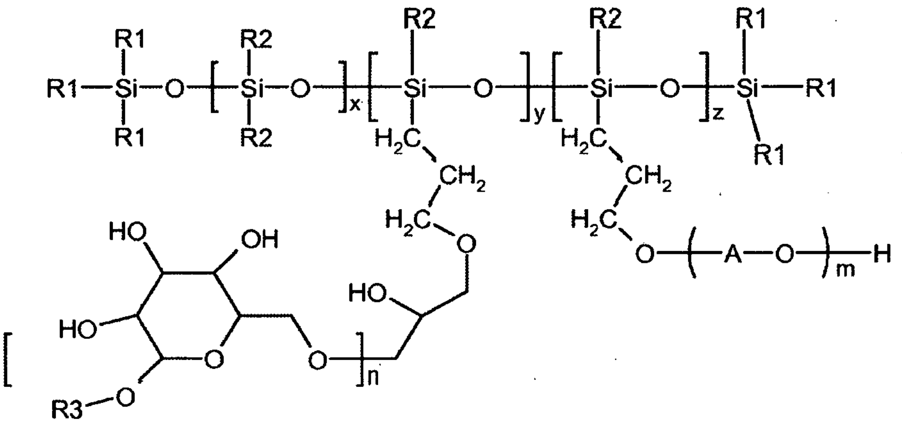

   in which A represents a group selected from $-CH_2CH_2-$, $-CH_2CH_2CH_2-$ or $-CH_2CH_2CH_2CH_2-$ or mixtures thereof, the functional groups R1, R2 and R3 independently of one another represent a methyl, ethyl, propyl, isopropyl, hydroxy, methoxy, or ethoxy group, x, y and z each represent an integer from 1 to 1000, and n and m independently of one another each represent an integer from 1 to 100, in a total amount of from 0.01 to 5.0 wt.%.

2. The cosmetic composition according to claim 1, **characterized in that**, in addition, at least one quaternary ammonium compound in a total amount of from 0.1 to 10.0 wt.%, based on the weight of the overall composition, selected from at least one of the groups of:

   i. esterquats and/or
   ii. quaternary imidazolines of formula (Tkat2),

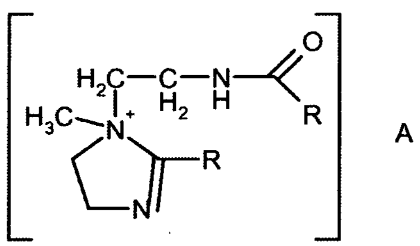

   in which the functional groups R independently of one another each represent a saturated or unsaturated, linear or branched hydrocarbon functional group having a chain length of 8 to 30 carbon atoms, and A represents a physiologically acceptable anion, and/or
   iii. amines and/or cationized amines and/or
   iv. poly(methacryloyloxyethyltrimethylammonium) compounds and/or

v. quaternized cellulose derivatives, in particular Polyquaternium-10, Polyquaternium-24, Polyquaternium-27, Polyquaternium-67, Polyquaternium-72, and/or
vi. cationic alkyl polyglycosides and/or
vii. cationized honey and/or
viii. cationic guar derivatives and/or
ix. chitosan and/or
x. polymeric dimethyldiallylammonium salts and copolymers thereof with esters and amides of acrylic acid and methacrylic acid, in particular Polyquaternium-7, and/or
xi. copolymers of vinylpyrrolidone with quaternized derivatives of dialkylaminoalkyl acrylate and dialkylaminoalkyl methacrylate, in particular Polyquaternium-11, and/or
xii. vinylpyrrolidone-vinylimidazolium methochloride copolymers, in particular Polyquaternium-16, and/or
xiii. quaternized polyvinyl alcohol, and/or
xiv. Polyquaternium-74,

and mixtures thereof.

**3.** The cosmetic composition according to the preceding claims, **characterized in that** the anti-dandruff agent is selected from zinc pyrithione and/or climbazole and/or Octopirox, and mixtures thereof.

**4.** The cosmetic composition according to the preceding claims, **characterized in that** the quaternary ammonium compound is selected from stearamidopropyl dimethylamine and/or distearoylethyl hydroxyethylmonium methosulfate and/or dicocoyl hydroxyethylmonium methosulfate and/or dipalmitoylethyl dimonium chloride and/or Quaternium-27 and/or Quaternium-91 and/or Behenoyl PG-Trimonium Chloride.

**5.** The cosmetic composition according to the preceding claims, **characterized in that** the sugar-structure-containing silicone is PEG-8 PG-Coco-Glucoside Dimethicone.

**6.** The cosmetic composition according to the preceding claims, **characterized in that** behenyl trimethylammonium chloride and/or cetyltrimethylammonium chloride is used as an additional cationic surfactant.

**7.** The cosmetic composition according to the preceding claims, **characterized in that**, in addition, at least one zwitterionic and/or amphoteric surfactant is contained.

**8.** The cosmetic composition according to the preceding claims, **characterized in that** the zwitterionic and/or amphoteric surfactant is selected from cocamidopropyl betaine and/or coco betaine.

**9.** The cosmetic composition according to the preceding claims, **characterized in that**, in addition, at least one amino acid selected from alanine, arginine, asparagine, aspartic acid, cysteine, cystine, citrulline, glutamic acid, glutamine, glycine, histidine, hydroxylysine, hydroxyproline, isoleucine, leucine, lysine, methionine, phenylalanine, proline, serine, threonine, thyroxine, tryptophan, tyrosine, acetyl tyrosine, valine, betaine, ornithine, 1,1-dimethyl proline, hercynine, ergothioneine and mixtures thereof is contained.

**10.** The cosmetic composition according to the preceding claims, **characterized in that**, in addition, at least one oligopeptide comprising at least 3 and no more than 25 amino acids. The cosmetic composition as set out above, **characterized in that**, in addition, at least one oligopeptide and containing the sequence Glu-Glu-Glu.

**11.** The cosmetic composition according to the preceding claims, **characterized in that**, in addition, at least one active ingredient selected from the group consisting of carnitine, taurine, coenzyme Q-10, ectoine, a purine and derivatives, in particular caffeine, or physiologically acceptable salts thereof, and a vitamin of the B series, in particular panthenol, is contained.

**12.** The cosmetic composition according to the preceding claims, **characterized in that**, in addition, at least one amino functional silicone is contained.

**13.** The cosmetic composition according to the preceding claims, **characterized in that** the amino functional silicone is selected from 4-morpholinomethyl-substituted silicones.

**14.** A method for treating keratin fibers, **characterized in that** a cosmetic composition according to the preceding claims

is applied to the keratin fibers and rinsed out again after an application time of from a few seconds up to 45 minutes.

**15.** The use of a cosmetic composition according to the preceding claims 1-13 for preventing the build-up of dandruff on the scalp.

**Revendications**

**1.** Composition cosmétique comprenant, dans un support cosmétique - respectivement rapporté au poids de la composition totale -

a) au moins un principe actif antipelliculaire en une quantité totale de 0,01 à 10,0 % en poids, choisi parmi

i. le pyrithione de zinc,
ii. le climbazole,
iii. l'octopirox,
iv. le kétoconazole,
v. le disulfure de sélénium,
vi. les huiles végétales contenant du sélénium et
vii. les extraits de plantes contenant du sélénium et leurs mélanges, et

b) au moins une silicone contenant des structures de sucre de la formule suivante,
dans laquelle A représente un groupe choisi parmi -CH2CH2-, -CH2CH2CH2- ou -

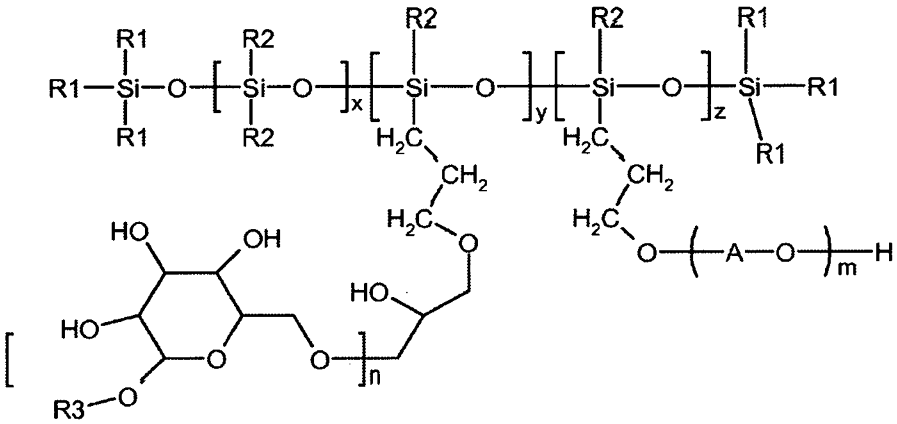

CH2CH2CH2CH2- ou des mélanges de ces groupes, les résidus R1, R2 et R3 représentent indépendamment l'un de l'autre un groupe méthyle, éthyle, propyle, iso-propyle, un groupe hydroxy, un groupe méthoxy ou un groupe éthoxy, x, y et z représentent respectivement un nombre entier de 1 à 1000, n et m représentent respectivement indépendamment l'un de l'autre un nombre entier de 1 à 100, en une quantité totale de 0,01 à 5,0 % en poids.

**2.** Composition cosmétique selon la revendication 1, **caractérisée en ce qu'**elle contient en outre au moins un composé d'ammonium quaternaire en une quantité totale de 0,1 à 10,0 % en poids, rapporté au poids de la composition totale, choisi parmi au moins l'un des groupes suivants

i. les esterquats et/ou
ii. les imidazolines quaternaires de formule (Tkat2),

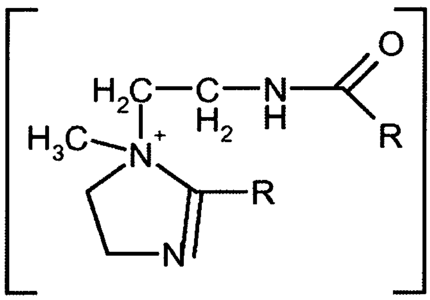

A

dans laquelle les résidus R indépendamment l'un de l'autre représentent respectivement un résidu hydrocarboné saturé ou insaturé, linéaire ou ramifié ayant une longueur de chaîne de 8 à 30 atomes de carbone et A représente un anion physiologiquement acceptable, et/ou

iii. des amines et/ou des amines cationisées et/ou

iv. des poly(composés de méthacryloyloxyéthyltriméthylammonium) et/ou ;

v. des dérivés quaternisés de la cellulose, notamment le polyquaternium 10, polyquaternium-24, polyquaternium-27, polyquaternium-67, polyquaternium-72, et/ou

vi. des alkylpolyglycosides cationiques et/ou

vii. du miel cationisé et/ou

viii. des dérivés de guar cationiques et/ou

ix. du chitosane et/ou

x. des sels polymères de diméthyldiallylammonium et leurs copolymères avec des esters et des amides de l'acide acrylique et de l'acide méthacrylique, notamment le polyquaternium-7 et/ou

xi. des copolymères de vinylpyrrolidone avec des dérivés de l'acrylate de dialkylaminoalkyle et le méthacrylate de dialkylaminoalkyle, notamment le polyquaternium-11, et/ou

xii. des copolymères de méthochlorure de vinylpyrrolidone-imidazolium vinyliques, et notamment le polyquaternium-16, et/ou

xiii. de l'alcool polyvinylique quaternisé et/ou

xiv. du polyquaternium-74,

et leurs mélanges.

3. Composition cosmétique selon les revendications précédentes, **caractérisée en ce que** l'agent antipelliculaire est choisi parmi la pyrithione de zinc et/ou le climbazole et/ou l'octopirox et leurs mélanges.

4. Composition cosmétique selon les revendications précédentes, **caractérisé en ce que** le composé d'ammonium quaternaire est choisi parmi les stéaramidopropyldiméthylamines et/ou le méthosulfate de distéaroyléthyl hydroxyéthylmonium et/ou le méthosulfate de dicocoyl hydroxyéthylmonium et/ou le chlorure dimonium dipalmitoyléthylique et/ou le quaternium-27 et/ou le quaternium-91 et/ou chlorure de PG-trimonium béhénoylique.

5. Composition cosmétique selon les revendications précédentes, **caractérisée en ce que** la silicone contenant des structures de sucre est le PEG-8 PG-Coco-Glucoside Dimethicone.

6. Composition cosmétique selon les revendications précédentes, **caractérisée en ce qu'**on utilise comme autre agent tensioactif cationique du chlorure de behentrimonium et/ou du chlorure de cétyltriméthylammonium.

7. Composition cosmétique selon les revendications précédentes, **caractérisée en ce qu'**elle contient en outre au moins un tensioactif zwitterionique et/ou amphotère.

8. Composition cosmétique selon les revendications précédentes, **caractérisée en ce que** le tensioactif zwitterionique et/ou amphotère est choisi parmi la bétaïne de cocamidopropyle et/ou la bétaïne de coco.

9. Composition cosmétique selon les revendications précédentes, **caractérisée en ce qu'**elle contient en outre au moins un acide aminé choisi parmi l'alanine, l'arginine, l'asparagine, l'acide aspartique, la cystéine, la cystine, la citrulline, l'acide glutamique, la glutamine, la glycine, l'histidine, l'hydroxylysine, l'hydroxyproline, l'isoleucine, la leucine, la lysine, la méthionine, la phénylalanine, la proline, la sérine, la thréonine, la thyroxine , le tryptophane, la tyrosine, l'acétyl tyrosine, la valine, la bétaïne, l'ornithine, la 1,1-diméthyl-proline, l'hercynine et l'ergothionéine, et

des mélanges de ceux-ci.

**10.** Composition cosmétique selon les revendications précédentes, **caractérisée en ce qu'**en outre au moins un oligopeptide comprenant au moins 3 à 25 acides aminés au maximum. Composition cosmétique telle que présentée ci-dessus, **caractérisée en ce qu'**en outre au moins un oligopeptide et contenant la séquence Glu-Glu-Glu.

**11.** Composition cosmétique selon les revendications précédentes, **caractérisée en ce qu'**elle contient en outre au moins un principe actif choisi dans le groupe constitué de la carnitine, la taurine, la coenzyme Q-10, l'ectoïne, d'une purine et ses dérivés, en particulier la caféine, ou de leurs sels physiologiquement acceptables, ainsi que d'une vitamine de type B, et en particulier le panthénol.

**12.** Composition cosmétique selon les revendications précédentes, **caractérisée en ce qu'**elle contient en outre au moins une silicone amino-fonctionnelle.

**13.** Composition cosmétique selon les revendications précédentes, **caractérisée en ce que** la silicone amino-fonctionnelle est choisie parmi les silicones de 4-morpholinométhyl substitués.

**14.** Procédé de traitement des fibres kératiniques, **caractérisé en ce qu'**une composition cosmétique est appliquée selon les revendications précédentes sur les fibres kératiniques puis rincée après un temps d'action allant de quelques secondes à 45 minutes.

**15.** Utilisation d'une composition cosmétique selon les revendications 1 à 13 précédentes pour la réduction de la formation de pellicules sur le cuir chevelu.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur

- International Cosmetic Ingredient Dictionary and Handbook. The Cosmetic, Toiletry, and Fragrance Association, 1997, vol. 1101, 300